# EUROPEAN PATENT APPLICATION

(11) **EP 4 234 660 A1**
(43) Date of publication of application: **30.08.2023**
(21) Application number: 22159007.8
(22) Date of filing: 25.02.2022
(51) Int. Cl.: C10G 7/00, B01D 3/14, C07C 7/04, C10G 45/58

(54) **A METHOD AND PLANT FOR ENERGY EFFICIENTLY PRODUCING N-HEXANE AND ISOMERATE HAVING A HIGH OCTANE NUMBER**

(71) Applicant: Sulzer Management AG, 8401 Winterthur (CH)
(72) Inventor: SEXANA, Alok Kumar, Ghaziabad-201009, UP (IN); SHAH, Ashishkumar Ashokkumar, Noida, 201301, UP (IN); MISRA, Himanshu, Gurgaon-122017, Haryana (IN); RAMANI, Prasanna, Houston, Texas 77077 (US)
(74) Representative: Henkel & Partner mbB

(57) **Abstract**

A method for producing n-hexane, which comprises the following steps:
a) producing an isomerate stream and preferably a stable isomerate stream in an isomerization unit comprising at least one isomerization reactor,
b) feeding the isomerate stream produced in step a) into a dividing-wall distillation column and distilling it,
c) removing from the dividing-wall distillation column four separate streams, namely a light isomerate stream, a heavy isomerate stream, a purified n-hexane stream and an isomerate process recycle stream and
d) recycling the isomerate process recycle stream to at least one of the at least one isomerization reactor of the isomerization unit.

## Description

The present invention relates to a method and to a plant of energy efficiently producing n-hexane and isomerate having a high octane number from a hydrocarbon feed, such as from an isomerate stream, such as from a stable C5-6 isomerate stream.

High-purity n-hexane is a light distillate product with a very narrow boiling range. It is used as a solvent in vegetable oil extraction processes, polymer processes and in the drug and pharmaceutical industries. A special boiling point ("SBP") product, usually consisting of hydrocarbons with between 5 and 10 carbon atoms and having a distillation range between 55 and 155°C, is also a light distillate used in the paint industry.

Traditionally, n-hexane and SBP product are produced by a solvent extraction process by carrying out extraction of a naphtha cut with an initial boiling point of for example 140°C using a solvent. The solvent and naphtha are fed to an extraction column, in which the solvent selectively extracts aromatics from the naphtha, thus producing a hydrocarbon stream with a low aromatics content called raffinate. The raffinate from the extraction column is fed to a raffinate wash column, in which the raffinate is washed with water in order to remove traces of solvent from the raffinate. The so obtained dearomatized naphtha is then treated in a mercaptan removal unit in order to remove sulfur compounds to thereby produce a dearomatized naphtha stream meeting the sulfur specification. The dearomatized naphtha stream is then fractionated in a series of three splitter columns in order to produce the desired hexane and SBP fractions or cuts, respectively. Even if such a solvent extraction process produces n-hexane, its quality is inferior. For example, the benzene and sulfur contents of n-hexane produced by the solvent extraction process are comparably high, with the benzene content being up to about 500 ppm wt. and the sulfur content being about 5 ppm wt., respectively.

Another known process uses the isomerization of the hydrocarbon stream. More specifically, a hydrocarbon feed stream, such as naphtha, is treated in a series of isomerization reactors, in which the aromatics contained in the hydrocarbon feed stream are saturated and the n-alkanes contained in the hydrocarbon feed stream are converted to iso-alkanes. Gas and liquid in the reactor effluent are separated in a product separator, wherein the liquid obtained in the product separator is routed to a stabilizer distillation column for stabilization thereof by removal of gas and liquefied petroleum gas (LPG) from the liquid. The stabilized isomerate is then split in a de-iso-hexanizer (DIH) distillation column so as to produce as overhead fraction a light isomerate stream, as bottom fraction a heavy isomerate stream and as side stream a stream, which is recycled into one of the isomerization reactors. This isomerization process produces only heavy and light isomerate streams as the desired product. However, instead of operating the DIH distillation column as described above, it is also known to produce a purified n-hexane stream from the stabilized isomerate produced as described above by using three distillation columns in series. In the first distillation column, which is operated as DIH distillation column, a light isomerate stream is obtained as overhead fraction, whereas the bottom fraction is fed into a second distillation column. The overhead fraction of the second distillation column is recycled into one of the isomerization reactors, whereas the bottom fraction of the second distillation column is fed into a third distillation column, in which a purified n-hexane stream is obtained as overhead fraction and a heavy isomerate stream is obtained as bottom fraction. However, this process has the disadvantage of being very energy intensive and requires high investment costs for the plurality of required distillation columns. International patent application WO2020/229892 A1 discloses a method of producing n-hexane as a byproduct from a C5-C6 isomerization unit, which comprises the steps i) of producing a stable isomerate feed in a C5-C6 isomerization unit, ii) of feeding the stable isomerate feed to the first side of a dividing wall column, the dividing wall column comprising a dividing wall that divides the dividing wall column at least partially into a first side and a second side, with one side of the first and second sides configured to operate as a deisohexanizer column and the other side of the first and second side configured to operate as a hexane column to produce a) a n-hexane stream, b) a light isomerate stream and c) a heavy isomerate stream, iii) of feeding the n-hexane and hydrogen to a mixer of a benzene hydrogenation unit connected with the dividing wall column to form a hexane-hydrogen mixture, iv) of preheating the hexane-hydrogen mixture, v) of feeding the preheated hexane-hydrogen mixture to a polishing reactor of the benzene hydrogenation unit, wherein the polishing reactor hydrogenates at least a part of the benzene included in the produced hexane and vi) of feeding an output stream from the polishing reactor to a stripper column for separating lights from the hexane, wherein the stripper column is arranged downstream of the hexane polishing reactor. However, this method has the disadvantage that the final isomerate, which is the sum of the light isomerate stream and of the heavy isomerate stream, has a comparable low octane number of at most 84.

In view of this, the object underlying the present invention is to provide a method and plant producing n-hexane in a high yield and isomerate having a comparable high octane number of more than 85 and preferably of at least 87, wherein the method is energy efficient and requires comparable low investment costs for the plant.

In accordance with the present invention this object is satisfied by providing a method for producing n-hexane, which comprises the following steps:
a) producing an isomerate stream (and preferably a stable isomerate stream) in an isomerization unit comprising at least one isomerization reactor,
b) feeding the isomerate stream produced in step a) into a dividing-wall distillation column and distilling it,
c) removing from the dividing-wall distillation column four separate streams, namely a light isomerate stream, a heavy isomerate stream, a purified n-hexane stream and an isomerate process recycle stream and
d) recycling the isomerate process recycle stream to at least one of the isomerization reactors of the isomerization unit.

This solution bases on the surprising finding that by using a dividing-wall distillation column being fed with an isomerate stream (and preferably a stable isomerate stream) having been produced in an isomerization unit comprising at least one isomerization reactor and by operating the dividing-wall distillation column so that four separate hydrocarbon streams are removed therefrom, namely a light isomerate stream, a heavy isomerate stream, a purified n-hexane stream and an isomerate process recycle stream, with the isomerate process recycle stream being recycled into at least one of the at least one isomerization reactor of the isomerization unit, not only pure n-hexane is produced with a high yield in an energy efficient manner only requiring comparable small investment costs for the plant, but that also the produced light isomerate stream as well as the produced heavy isomerate stream have a comparable high octane number. More specifically, the total isomerate stream, i.e. the sum of the light isomerate stream and of the heavy isomerate stream, has an octane number (RON) of 87 to 89. The method in accordance with the present invention requires up to 45% less energy and less equipment with the same separation efficiency than the aforementioned process using three distillation columns in series for producing a n-hexane stream, a light isomerate stream and a heavy isomerate stream from a (preferably stable) isomerate stream produced in an isomerization unit from naphtha. The use of the dividing-wall column significantly improves the viability of this method by allowing the separation to take place in the same distillation column by avoiding back mixing of the heaviest components with the middle boiling components. Due to the segregation of the distillation column, an adequate number of trays or theoretical stages, respectively, are available on each side of dividing wall of the distillation column so as to facilitate an efficient separation of the components. Hence, a dividing-wall distillation column is quite profitable for high-purity hexane production as by product from an isomerization process. The removal of an isomerate process recycle stream from the divided-wall distillation column in addition to a light isomerate stream, to a heavy isomerate stream and to a purified n-hexane stream and the recycling of the isomerate process recycle stream into at least one of the isomerization reactor of the isomerization unit, i.e. the recycle of low octane components, such as 2-methyl pentane and 3-methyl pentane, allows to significantly increase the octane number of the produced light and heavy isomerate streams.

The isomerate stream produced in step a) may be an unstable isomerate stream obtained in a C4-7 isomerization unit, in a C5-7 isomerization unit, in a C4-6 isomerization unit or in a C5-6 isomerization unit. However, it is particularly preferred in the present patent application that the isomerate stream produced in step a) is a stable isomerate stream. Preferably, the stable isomerate stream is obtained in a C4-7 isomerization unit, in a C5-7 isomerization unit, in a C4-6 isomerization unit or in a C5-6 isomerization unit. Stable isomerate stream means in accordance with the present invention that the lighter boiling compounds, such as in particular C4 and lighters [i.e. C4 minus], have been almost completely removed from the isomerate. Thus, the bottoms stream from a stabilzer column is stabilized isomerate.

Preferably, the isomerate stream produced in step a) comprises at least 80 wt% of C4-7 hydrocarbons, more preferably at least 90 wt% of C4-7 hydrocarbons and most preferably at least 95 wt% of C4-7 hydrocarbons. It is preferred that at least 50 wt%, more preferably at least 60 wt% and most preferably at least 70 wt% of the isomerate stream produced in step a) are branched alkanes. Apart from the branched alkanes, n-alkanes, such as n-pentane and/or n-hexane, may be contained in the isomerate stream produced in step a), preferably in an amount of 5 to 30 wt% and more preferably in an amount of 10 to 20 wt%. In addition, the isomerate stream produced in step a) may comprise cycloalkanes or naphthenes, respectively, preferably in an amount of 1 to 20 wt% and more preferably in an amount of 5 to 15 wt%. Also, C6 aromatics (benzene) may be contained in trace amounts in the isomerate stream produced in step a), preferably in amounts of up to 100 ppm wt and more preferably in amounts of up to 50 ppm wt. The total amount of C3- hydrocarbons and of C7+ hydrocarbons preferably amounts to less than 20 wt%, more preferably to less than 10 wt% and most preferably to less than 5 wt%.

Alternatively, the isomerate stream produced in step a) comprises at least 80 wt% of C5-6 hydrocarbons, more preferably at least 90 wt% of C5-6 hydrocarbons and most 95 wt% of C5-6 hydrocarbons. Also in this embodiment it is preferred that at least 50 wt%, more preferably at least 60 wt% and most preferably at least 70 wt% of the isomerate stream produced in step a) are branched alkanes. Apart from the branched alkanes, n-alkanes, such as n-pentane and/or n-hexane, may be contained in the isomerate stream produced in step a), preferably in an amount of 5 to 30 wt% and more preferably in an amount of 10 to 20 wt%. In addition, the isomerate stream produced in step a) may comprise cycloalkanes or naphthenes, respectively, preferably in an amount of 1 to 20 wt% and more preferably in an amount of 5 to 15 wt%. Also, aromatics may be contained in the isomerate stream produced in step a), preferably in amounts of up to 100 ppm wt and more preferably in amounts of up to 50 ppm wt. The total amount of C4- [i.e. C4 minus] hydrocarbons and of C6+ hydrocarbons preferably amounts to less than 20 wt%, more preferably to less than 10 wt% and most preferably to less than 5 wt%.

In accordance with a further particular preferred embodiment of the present invention, the purified n-hexane stream removed in step c) from the dividing-wall distillation column has a n-hexane content of at least 30 wt%. More preferably, the purified n-hexane stream has a n-hexane content of at least 35 wt%, yet more preferably of 35 to 45 wt%, still more preferably of at least 40 wt% and most preferably of 40 to 45 wt%. In addition to the n-hexane, other C6 hydrocarbon compounds may be contained in the purified n-hexane stream, such as C6 iso-alkanes and C6 cycloalkanes. For instance, the purified n-hexane stream contains 20 to 50 wt% of C6 iso-alkanes and 5 to 30 wt% C6 cycloalkanes.

In a further development of the idea of the present invention, it is suggested that the purified n-hexane stream removed in step c) from the dividing-wall distillation column has a benzene content of < 3 ppm wt. and a sulfur content of < 0.5 ppm wt.

In a further development of the idea of the present invention, it is suggested that the purified n-hexane stream removed in step c) from the dividing-wall distillation column has an initial boiling point (IBP) of 63°C minimum and 95% by volume distilled between 64 to 70°C per ASTM D86 test method.

The light isomerate stream removed in step c) from the dividing-wall distillation column is mainly composed of C6- [i.e. C6 minus] hydrocarbons and preferably comprises at least 80 wt% and more preferably at least 90 wt% of C6- [i.e. C6 minus] hydrocarbons. Moreover, it preferably comprises at least 80 wt% of branched C5-6 hydrocarbons, wherein preferably at least 70 wt%, more preferably at least 80 wt% and most preferably at least 90 wt% of the light isomerate stream are branched alkanes. It is further preferred that the light isomerate stream comprises at least 30 wt%, more preferably at least 40 wt% and most preferably at least 50 wt% of double branched C6 alkanes, such as 2,2-methylbutane and/or 2,3-methylbutane. For instance, the light isomerate stream comprises 20 to 60 wt% of C5 hydrocarbons and 40 to 80 wt% of C6 hydrocarbons. More specifically, the light isomerate stream may comprise 20 to 40 wt% of C5-iso-alkanes, 40 to 80 wt% of C6 iso-alkanes (preferably 40 to 60 wt% of double branched C6 alkanes and up to 20 wt% of single branched C6 alkanes) and up to 20 wt% of C6 n-alkanes. It is particularly preferred that the light isomerate stream removed in step c) from the dividing-wall distillation column has an octane number of 87 to 89.

In accordance with a further particular preferred embodiment of the present invention, the heavy isomerate stream removed in step c) from the dividing-wall distillation column comprises at least 80 wt% of C6+ hydrocarbons and preferably at least 40 wt%, more preferably at least 50 wt% and most preferably at least 60 wt% of C7+ hydrocarbons. Moreover, it preferably comprises 10 to 60 wt%, more preferably 20 to 50 wt% and most preferably 20 to 40 wt% of C6- cycloalkanes. Preferably, at least 40 wt%, more preferably at least 50 wt% and most preferably at least 60 wt% of the heavy isomerate stream are cycloalkanes. For instance, the heavy isomerate stream comprises 20 to 40 wt% of C6 cycloalkanes, 20 to 40 wt% of C7 n-alkanes and 30 to 50 wt% of C7 cycloalkanes. It is particularly preferred that the heavy isomerate stream removed in step c) from the dividing-wall distillation column has an octane number of 82 to 87.

The light isomerate stream and the heavy isomerate stream removed in step c) from the dividing-wall distillation column may be combined to a total isomerate stream. Independently, from whether the light isomerate stream and the heavy isomerate stream removed in step c) from the dividing-wall distillation column are combined to a total isomerate stream, the sum of the light isomerate stream and of the heavy isomerate stream removed in step c) from the dividing-wall distillation column preferably has an octane number of 87 to 89. Octane number means in the present invention the RON ("research ***octane*** number") (ASTM D 2699).

In a further development of the idea of the present invention, it is suggested that the light isomerate stream removed in step c) from the dividing-wall distillation column comprises at least 50 wt%, preferably at least 60 wt%, more preferably at least 70 wt%, yet more preferably at least 80 wt% and most preferably at least 90 wt% of double branched C6 alkanes, such as 2,2-methylbutane and 2,3-methylbutane. Single branched C6 alkanes, such as 2-methylpentane and 3-methylpentane, may be contained in the isomerate process recycle stream in amounts of up to 75 wt%, such as in amounts of 20 to 75 wt%, of 25 to 60 wt% or of 30 to 50 wt %.

The present invention is not particularly limited concerning the type of dividing-wall distillation column used in steps b) and c). Thus, the dividing-wall distillation column may be a top dividing-wall distillation column, a middle dividing-wall distillation column or a bottom dividing-wall distillation column.

In accordance with a first particular preferred embodiment of the present invention, the dividing-wall distillation column used in steps b) and c) is a top dividing-wall column.

Good results are obtained in a first variant of the first particular preferred embodiment of the present invention, when the dividing wall of the top dividing-wall column extends from the upper end of the top dividing-wall column over 20 to 80% and preferably over 20 to 70% of the height of the top dividing-wall column at least essentially vertically downwards. Thus, the top dividing-wall distillation column comprises on one side of the dividing wall a first top section, on the opposite side of the dividing wall a second top section and below the dividing wall a bottom section. Essentially vertically downwards again means in accordance with the present invention that the angle between the dividing wall and the length axis of the top dividing-wall distillation column is at most 20°, preferably at most 10°, more preferably at most 5° and most preferably 0°. Preferably, the overhead gauge pressure of each of the two top sections is independently controlled and maintained via a pressure controller.

In a further development of the idea of the present invention, it is proposed that the top dividing-wall distillation column is operated so that it has a height to accommodate 70 to 160 theoretical stages. Preferably, the first top section of the top dividing-wall distillation columns comprises 20 to 50 theoretical stages, the second top section of the top dividing-wall distillation columns comprises 20 to 50 theoretical stages and the bottom section comprises 30 to 60 theoretical stages.

Preferably, the isomerate stream is fed in step b) into the first top section of the top dividing-wall distillation column, wherein in step c) the light isomerate stream is removed as overhead fraction from the first top section of the top dividing-wall distillation column, the purified n-hexane stream is removed as overhead fraction from the second top section of the top dividing-wall distillation column, the heavy isomerate stream is removed as bottom fraction from the bottom section of the top dividing-wall distillation column and the isomerate process recycle stream is removed as side fraction from the first top section of the top dividing-wall distillation column.

Moreover, it is preferred in this embodiment that at least one, preferably at least two and most preferably all of the subsequent are fulfilled:
i) The light isomerate stream removed from the top dividing-wall distillation column in step c) is at least partially condensed in an overhead condenser (preferably in an air-cooled heat exchanger), wherein a part of the condensed light isomerate stream is recycled into the first top section of the top dividing-wall distillation column, wherein the reflux ratio is 3 to 10 and wherein preferably the gauge pressure in the top dividing-wall distillation column is 0.05 to 0.3 MPa, and/or
ii) the purified n-hexane stream removed from the top dividing-wall distillation column in step c) is at least partially condensed in an overhead condenser (preferably in an air-cooled heat exchanger), wherein a part of the condensed purified n-hexane stream is recycled into the second top section of the top dividing-wall distillation column, wherein the reflux ratio is 6 to 15 and wherein preferably the gauge pressure in the top dividing-wall distillation column is 0.05 to 0.3 MPa, and/or
iii) the heavy isomerate stream removed from the top dividing-wall distillation column bottom in step c) is reboiled in a bottom reboiler wherein a part of the reboiled heavy isomerate stream is recycled into the bottom section of the top dividing-wall distillation column, wherein sufficient re-boiling is done to establish required vapor - liquid traffic and required ratio(s) in top dividing wall column, while heavy isomerate product is withdrawn under level control of top dividing wall column bottom, and wherein preferably the gauge pressure in the top dividing-wall distillation column is 0.05 to 0.3 MPa.

Moreover, the temperature in the top sections of the top dividing-wall distillation column is cascaded to the reflux flow control loops on each side of the dividing wall to allow control over the quality of the products. This control philosophy prevents the heavier components from going to the top of the column. This allows the control of n-hexane product quality with respect of final boiling point / cyclohexane and minimize single branch C6 range i-paraffins slippage to light isomerate. The re-boiling of the dividing-wall distillation column is controlled by steam flow to reboilers cascade with column bottom temperature. The heavy isomerate product flow rate from the of the top dividing-wall distillation column is controlled by cascading with a level control loop in the lower section.

In a second variant of the first particular preferred embodiment of the present invention, in which the dividing-wall column used in steps b) and c) is a top dividing-wall column, the dividing wall of the top dividing-wall column extends from the upper end of the top dividing-wall column over 5 to 60% and preferably over 10 to 50% of the height of the top dividing-wall column at least essentially vertically downwards, wherein the top dividing-wall column further comprises a lower partition wall arranged below the dividing wall. The partition wall preferably comprises an essentially horizontally arranged section and a lower essentially vertically arranged section, wherein the upper essentially horizontally arranged section comprises a first edge and a second edge and the lower essentially vertically arranged section comprises an upper edge and a lower edge. The upper edge of the lower essentially vertically arranged section and the first edge of the upper essentially horizontally arranged section of the partition wall are connected with each other over the whole length of both edges, wherein the second edge of the upper essentially horizontally arranged section of the partition wall is fluid tightly connected with the outer wall of the top dividing-wall column. Essentially vertically downwards again means that the angle between the dividing wall and the length axis of the top dividing-wall distillation column is at most 20°, preferably at most 10°, more preferably at most 5° and most preferably 0°, wherein essentially horizontally means that the angle between the dividing wall and the cross-sectional plane of the top dividing-wall distillation column is at most 20°, preferably at most 10°, more preferably at most 5° and most preferably 0°. Thus, the top dividing-wall distillation column of this second variant of the first particularly preferred embodiment of the present invention comprises on one side of the dividing wall a first top section, on the opposite side of the dividing wall a second top section, in the volume below the essentially horizontally arranged section of the partition wall extending until the lower edge of the essentially vertically arranged section of the partition wall a partitioned section (working as further rectifying section) and in the residual volume of the top dividing-wall column a bottom section. Hence, the partition wall does not allow the descending liquid to enter the partitioned section through the partition wall and also does not allow the ascending vapor to leave the partitioned section through the partition wall. Preferably, the overhead gauge pressure of each of the two top sections as well as the gauge pressure of the partitioned section are independently controlled and maintained via a pressure controller.

Good results are in particular obtained, when the top dividing-wall distillation column is operated in this second variant of the first particular preferred embodiment of the present invention so that it has a height to accommodate 80 to 205 theoretical stages, wherein preferably the first top section of the top dividing-wall distillation columns comprises 20 to 50 theoretical stages, the second top section of the top dividing-wall distillation columns comprises 20 to 50 theoretical stages, the partitioned section of the top dividing-wall distillation columns comprises 10 to 25 theoretical stages and the bottom section comprises 30 to 80 theoretical stages.

Preferably, the isomerate stream is fed in step b) into the first top section of the top dividing-wall distillation column, wherein in step c) the light isomerate stream is removed as overhead fraction from the first top section of the top dividing-wall distillation column, the isomerate process recycle stream is removed as overhead fraction from the second top section of the top dividing-wall distillation column, the heavy isomerate stream is removed as bottom fraction from the bottom section of the top dividing-wall distillation column and the purified n-hexane stream is removed as side fraction from the lower partitioned section of the top dividing-wall distillation column.

Furthermore, it is preferred in this embodiment that at least one, preferably at least two and most preferably all of the subsequent are fulfilled:
i) The light isomerate stream removed from the top dividing-wall distillation column in step c) is at least partially condensed in an overhead condenser (preferably in an air-cooled heat exchanger), wherein a part of the condensed light isomerate stream is recycled into the first top section of the top dividing-wall distillation column, wherein the reflux ratio is 3 to 10 and wherein preferably the gauge pressure in the top dividing-wall distillation column is 0.05 to 0.3 MPa, and/or
ii) the isomerate process recycle stream is removed from the top dividing-wall distillation column in step c) is at least partially condensed in an overhead condenser (preferably in an air-cooled heat exchanger), wherein a part of the condensed isomerate process recycle stream is recycled into the second top section of the top dividing-wall distillation column, wherein the reflux ratio is 2 to 6, and wherein preferably the gauge pressure in the top dividing-wall distillation column is 0.05 to 0.3 MPa, and/or
iii) the heavy isomerate stream removed from the top dividing-wall distillation column in step c) is reboiled in a bottom reboiler, wherein a part of the heavy reboiled isomerate stream is recycled into the bottom section of the top dividing-wall distillation column, wherein sufficient re-boiling is done to establish required vapor - liquid traffic and required ratio(s) in top dividing wall column, while heavy isomerate product is withdrawn under level control of top dividing wall column bottom and wherein preferably the gauge pressure in the top dividing-wall distillation column is 0.05 to 0.3 MPa, and/or
iv) the purified n-hexane stream removed from the top dividing-wall distillation column in step c) is at least partially condensed in a side condenser (preferably in an air-cooled heat exchanger), wherein a part of the condensed purified n-hexane stream is recycled into the lower partitioned section of the top dividing-wall distillation column, wherein the reflux ratio is 0.5 to 4 and wherein preferably the gauge pressure in the top dividing-wall distillation column is 0.05 to 0.3 MPa.

Moreover, the temperature in the top sections of the top dividing-wall distillation column is cascaded to the reflux flow control loop to allow control over the quality of the product. This control philosophy prevents the heavier components from going to the top of the column. Similarly, temperature/differential temperature in the lower partitioned section is cascaded to the reflux flow from the receiver of this partitioned section. This allows the control of the n-hexane product quality with respect of final boiling point and cyclohexane. The re-boiling of the dividing-wall distillation column is controlled by steam flow to reboilers cascade with column bottom temperature. The heavy isomerate product flow rate from the of the top dividing-wall distillation column is controlled by cascading with a level control loop in the lower section.

In accordance with a second particular preferred embodiment of the present invention, the dividing-wall distillation column used in steps b) and c) is a bottom dividing-wall column, wherein preferably the dividing wall of the bottom dividing-wall column extends from the lower end of the bottom dividing-wall column over 10 to 60% and preferably over 20 to 50% of the height of the bottom dividing-wall column at least essentially vertically upwards. Thus, the bottom dividing-wall distillation column comprises on one side of the dividing wall a first bottom section, on the opposite side of the dividing wall a second bottom section and above the dividing wall a top section, wherein essentially vertically upwards means that the angle between the dividing wall and the length axis of the bottom dividing-wall distillation column is at most 20°, preferably at most 10°, more preferably at most 5° and most preferably 0°.

Good results are in particular obtained, when the bottom dividing-wall distillation column is operated so that it has a height to accommodate 70 to 180 theoretical stages, wherein preferably the first bottom section of the bottom dividing-wall distillation columns comprises 20 to 60 theoretical stages, the second bottom section of the middle dividing-wall distillation columns comprises 20 to 60 theoretical stages and the top section comprises 30 to 60 theoretical stages.

Preferably, the isomerate stream is fed in step b) into the first bottom section of the bottom dividing-wall distillation column, wherein in step c) the light isomerate stream is removed as overhead fraction from the top section of the bottom dividing-wall distillation column, the heavy isomerate stream is removed as bottom fraction from the first bottom section of the middle dividing-wall distillation column, the purified n-hexane stream is removed as bottom fraction from the second bottom section of the middle dividing-wall distillation column and the isomerate process recycle stream is removed as side fraction from the second bottom section of the bottom dividing-wall distillation column.

In addition, it is preferred in this embodiment that at least one, preferably at least two and most preferably all of the subsequent are fulfilled:
i) The light isomerate stream removed from the top of the bottom dividing-wall distillation column in step c) is at least partially condensed in an overhead condenser (preferably in an air-cooled heat exchanger), wherein a part of the condensed light isomerate stream is recycled into the top section of the bottom dividing-wall distillation column, wherein the reflux ratio is 3 to 12 and wherein preferably the gauge pressure in the middle dividing-wall distillation column is 0.05 to 0.3 MPa, and/or
ii) the heavy isomerate stream removed from the bottom dividing-wall distillation column first section bottom in step c) is reboiled in a bottom reboiler, wherein a part of the reboiled heavy isomerate stream is recycled into the first bottom section of the bottom dividing-wall distillation column, wherein sufficient re-boiling is done to establish required vapor - liquid traffic and required reflux ration in bottom dividing wall column, while heavy isomerate product is withdrawn under level control of top dividing wall column bottom and wherein preferably the gauge pressure in the bottom dividing-wall distillation column is 0.05 to 0.3 MPa, and/or
iii) the purified n-hexane stream removed from the bottom dividing-wall distillation column second bottom section in step c) is reboiled in a bottom reboiler, wherein a part of the reboiled purified n-hexane stream is recycled into the second bottom section of the bottom dividing-wall distillation column, wherein sufficient re-boiling is done to establish required vapor - liquid traffic and required reflux ratio in bottom dividing wall column, while purified n-hexane product is withdrawn under level control of top dividing wall column bottom and wherein preferably the gauge pressure in the bottom dividing-wall distillation column is 0.05 to 0.3MPa.

Moreover, the temperature on each side of the bottom sections of the bottom dividing-wall distillation column is cascaded to the steam flow to the reboilers on each side respectively to allow control over the quality of the product. This control philosophy prevents the heavier components from going to the top of the column from first and second bottom sections of bottom dividing wall column and maintain products quality. This allows the control of the n-hexane product quality with respect of final boiling point and cyclohexane. The heavy isomerate and n-hexane products flow rate from the of the bottom dividing-wall distillation column is controlled by cascading with a level control loops on each side of the bottom dividing wall in the lower section.

In accordance with a third particular preferred embodiment of the present invention, the dividing-wall distillation column used in steps b) and c) is a middle dividing-wall column.

Good results are in a first variant of the third particular preferred embodiment of the present invention obtained, when the dividing wall of the middle dividing-wall column extends, seen from the bottom to the top of the middle dividing-wall distillation column, from a point being located at 20 to 50% of the distance from the bottom to the top of the middle dividing-wall distillation column to a point being located at 70 to 90% of the distance from the bottom to the top of the middle dividing-wall distillation column at least essentially vertically downwards. Thus, the middle dividing-wall distillation column comprises above the dividing wall a top section, below the dividing wall a bottom section, on one side of the dividing wall a first middle section and on the opposite side of the dividing wall a second middle section. Essentially vertically downwards again means that the angle between the dividing wall and the length axis of the middle dividing-wall distillation column is at most 20°, preferably at most 10°, more preferably at most 5° and most preferably 0°.

Moreover, it is preferred that the dividing wall extends over 20 to 80%, preferably over 30 to 70% and more preferably over 30 to 60% of the height of the middle dividing-wall distillation column, wherein the height of the middle dividing-wall distillation column is the straight distance between the top and the bottom of the middle dividing-wall distillation column.

Good results are in particular obtained, when the middle dividing-wall distillation column is operated so that it has a height to accommodate 60 to 180 theoretical stages, wherein preferably the top section of the top dividing-wall distillation columns comprises 10 to 30 theoretical stages, the first middle section of the top dividing-wall distillation columns comprises 20 to 60 theoretical stages, the second middle section of the top dividing-wall distillation columns comprises 20 to 60 theoretical stages and the bottom section comprises 10 to 30 theoretical stages.

Preferably, the (preferably stable) isomerate stream is fed in step b) into the first middle section of the middle dividing-wall distillation column, wherein in step c) the light isomerate stream is removed as overhead fraction from the top section of the middle dividing-wall distillation column, the isomerate process recycle stream is removed as side fraction from the second middle section of the middle dividing-wall distillation column, the purified n-hexane stream is removed as side fraction from the second middle section of the middle dividing-wall distillation column and the heavy isomerate stream is removed as bottom fraction from the bottom section of the top dividing-wall distillation column. Preferably, the purified n-hexane stream is removed as side fraction from a point being below the point, from which the process recycle stream is removed as side fraction from the second middle section of the middle dividing-wall distillation column. Good results are in particular obtained, when the purified n-hexane stream is removed as side fraction from a point being 20 to 50% of the height of the middle dividing-wall distillation column below the point, from which the process recycle stream is removed as side fraction from the second middle section of the middle dividing-wall distillation column. Furthermore, it is preferred in this embodiment that at least one, preferably at least two and most preferably all of the subsequent are fulfilled:
i) The light isomerate stream removed from the top dividing-wall distillation column in step c) is at least partially condensed in an overhead condenser (preferably in an air-cooled heat exchanger), wherein a part of the condensed light isomerate stream is recycled into the top section of the middle dividing-wall distillation column, wherein the reflux ratio is 3 to 12 and wherein preferably the gauge pressure in the top dividing-wall distillation column is 0.05 to 0.3 MPa, and/or
ii) the heavy isomerate stream removed from the bottom of the middle dividing-wall distillation column in step c) is reboiled in a bottom reboiler, wherein a part of the heavy reboiled isomerate stream is recycled into the bottom section of the middle dividing-wall distillation column, wherein sufficient re-boiling is done to establish required vapor - liquid traffic and required reflux ratio in middle dividing wall column, while heavy isomerate product is withdrawn under level control of top dividing wall column bottom and wherein preferably the gauge pressure in the middle dividing-wall distillation column is 0.05 to 0.3 MPa.

Moreover, the temperature in the top section of the middle dividing-wall distillation column is cascaded to the reflux flow control loop to allow control over the quality of the product. This control philosophy prevents the heavier components from going to the top of the column. This allows the control of the n-hexane product quality with respect of final boiling point and cyclohexane. The re-boiling of the middle dividing-wall distillation column is controlled by steam flow to reboilers cascade with column bottom temperature. The heavy isomerate product flow rate from the of the middle dividing-wall distillation column is controlled by cascading with a level control loop in the lower section.

In a second variant of the third particular preferred embodiment of the present invention, in which the dividing-wall column used in steps b) and c) is a middle dividing-wall column, the dividing wall of the middle dividing-wall column extends, seen from the bottom to the top of the middle dividing-wall distillation column, from a point being located at 40 to 60% of the distance from the bottom to the top of the middle dividing-wall distillation column to a point being located at 70 to 90% of the distance from the bottom to the top of the middle dividing-wall distillation column at least essentially vertically downwards, wherein the middle dividing-wall column further comprises a partition wall arranged below the middle dividing wall. The partition wall comprises an essentially horizontally arranged section and a lower essentially vertically arranged section, wherein the upper essentially horizontally arranged section comprises a first edge and a second edge and the lower essentially vertically arranged section comprises an upper edge and a lower edge. The upper edge of the lower essentially vertically arranged section and the first edge of the upper essentially horizontally arranged section of the partition wall are connected with each other over the whole length of both edges, wherein the second edge of the upper essentially horizontally arranged section of the partition wall is fluid tightly connected with the outer wall of the top dividing-wall column. Again, essentially vertically downwards means that the angle between the dividing wall and the length axis of the middle dividing-wall distillation column is at most 20°, preferably at most 10°, more preferably at most 5° and most preferably 0°, wherein essentially horizontally means that the angle between the dividing wall and the cross-sectional plane of the middle dividing-wall distillation column is at most 20°, preferably at most 10°, more preferably at most 5° and most preferably 0°. Thus, the middle dividing-wall distillation column comprises above the dividing wall a top section, on one side of the dividing wall a first middle section, on the opposite side of the dividing wall a second middle section, in the volume below the essentially horizontally arranged section of the partition wall extending until the lower edge of the essentially vertically arranged section of the partition wall a partitioned section (working as further rectifying section) and in the residual volume of the middle dividing-wall column a bottom section. Hence, the partition wall does not allow the descending liquid to enter the partitioned section through the partition wall and also does not allow the ascending vapor to leave the partitioned section through the partition wall.

Good results are in particular obtained, when the middle dividing-wall distillation column is operated so that it has a height to accommodate 58 to 165 theoretical stages, wherein preferably the top section of the middle dividing-wall distillation columns comprises 8 to 30 theoretical stages, the first middle section of the middle dividing-wall distillation columns comprises 15 to 40 theoretical stages, the second middle section of the middle dividing-wall distillation columns comprises 15 to 40 theoretical stages, the lower partitioned section of the top dividing-wall distillation columns comprises 10 to 25 theoretical stages and the bottom section comprises 10 to 30 theoretical stages.

Preferably, the isomerate stream is fed in step b) into the first middle section of the middle dividing-wall distillation column, wherein in step c) the light isomerate stream is removed as overhead fraction from the top section of the middle dividing-wall distillation column, the isomerate process recycle stream is removed as side fraction from the second middle section of the middle dividing-wall distillation column, the purified n-hexane stream is removed as side fraction from the lower partitioned section of the middle dividing-wall distillation column and the heavy isomerate stream is removed as bottom fraction from the bottom section of the middle dividing-wall distillation column. Preferably, the purified n-hexane stream is removed as side fraction from lower partitioned section, a point being below the point, from which the process recycle stream is removed as side fraction from the second middle section of the middle dividing-wall distillation column. Good results are in particular obtained, when the purified n-hexane stream is removed as side fraction from a point being 20 to 50% of the height of the middle dividing-wall distillation column below the point, from which the process recycle stream is removed as side fraction from the second middle section of the middle dividing-wall distillation column.

In addition, it is preferred in this embodiment that at least one, preferably at least two and most preferably all of the subsequent are fulfilled:
i) The light isomerate stream removed from the middle dividing-wall distillation column in step c) is at least partially condensed in an overhead condenser (preferably in an air-cooled heat exchanger), wherein a part of the condensed light isomerate stream is recycled into the top section of the middle dividing-wall distillation column, wherein the reflux ratio is 3 to 10 and wherein preferably the gauge pressure in the middle dividing-wall distillation column is 0.05 to 0.3 MPa, and/or
ii) the purified n-hexane stream removed from the middle dividing-wall distillation column in step c) is at least partially condensed in a side condenser (preferably in an air-cooled heat exchanger), wherein a part of the condensed purified n-hexane stream is recycled into the partitioned section of the middle dividing-wall distillation column, wherein the reflux ratio is 0.5 to 3 and wherein preferably the gauge pressure in the middle dividing-wall distillation column is 0.05 to 0.3 MPa, and/or
iii) the heavy isomerate stream removed from the middle dividing-wall distillation column in step c) is reboiled in a bottom reboiler, wherein a part of the reboiled heavy isomerate stream is recycled into the bottom section of the middle dividing-wall distillation column, wherein sufficient re-boiling is done to establish required vapor - liquid traffic and required reflux ratio in middle dividing wall column, while heavy isomerate product is withdrawn under level control of top dividing wall column bottom and wherein preferably the gauge pressure in the middle dividing-wall distillation column is 0.05 to 3.0 MPa.

Moreover, the temperature in the top section of the middle dividing-wall distillation column is cascaded to the reflux flow control loop to allow control over the quality of the product. This control philosophy prevents the heavier components from going to the top of the column. Similarly, temperature/differential temperature in the below partitioned section is cascaded to the reflux flow from the receiver of this partitioned section. This allows the control of the n-hexane product quality with respect of final boiling point and cyclohexane. The re-boiling of the middle dividing-wall distillation column is controlled by steam flow to reboilers cascade with column bottom temperature. The heavy isomerate product flow rate from the of the middle dividing-wall distillation column is controlled by cascading with a level control loop in the lower section.

Preferably, the (preferably stable) isomerate stream is produced in step a) in an isomerization unit comprising one or two isomerization reactors. In case of two isomerization reactors, both isomerization reactors are preferably connected in series, i.e. preferably the outlet of the upstream isomerization reactor is connected with the inlet of the second, downstream isomerization reactor. The isomerization reactor(s) comprise(s) the catalyst(s) necessary for the isomerization reaction, i.e. for isomerizing n-alkanes to iso-alkanes. Suitable examples for such catalysts are zeolite based (Pt-Zeolite) catalysts, chlorinated alumina based (Pt-Al₂O₃-Cl) catalysts and mixed oxide based (Pt-MO₂SO₄; Pt-ZrO₂M_{X}O_{Y}SO₄) catalysts. The isomerization reactor(s) are preferably operated at a temperature of 110 to 260°C depending upon catalyst type and age and at a gauge pressure of 2.5 to 4.0 MPa. The feed fed preferably together with hydrogen into the isomerization reactor or, in case of two subsequent isomerization reactors, into the first upstream isomerization reactor may be naphtha. However, the feed or naphtha may be processed, before it is fed into the isomerization reactor. For instance, the naphtha may be first subjected to a first distillation step in a first distillation column so as to remove as overhead fraction light naphtha, whereas as bottom fraction heavy naphtha is obtained, which is fed into a second distillation column. The heavy naphtha is distilled in the second distillation column so as to obtain as overhead fraction an iso-pentane rich fraction and as bottom fraction a C5-6-rich hydrocarbon fraction, which is fed into the isomerization reactor or, in case of two subsequent isomerization reactors, into the first upstream isomerization reactor.

The outlet of the one isomerization reactor or, in case of two subsequent isomerization reactors, the outlet of the second, downstream isomerization reactor is preferably connected with the inlet of a distillation column, which is preferably a non-dividing-wall distillation column. In the distillation column, remaining lights, i.e. C4- [i.e. C4 minus] hydrocarbons are at least essentially completely removed from the isomerate stream obtained in the isomerization reactor(s) as overhead fraction so that a (preferably stable) isomerate stream is obtained as bottom fraction of this distillation column as the isomerate stream to be fed into the dividing-wall distillation column in step b).

As set out above, in step d) the isomerate process recycle stream is recycled (preferably directly recycled) into at least one of the at least one isomerization reactor of the isomerization unit. In case of one isomerization reactor, the isomerate process recycle stream is recycled (preferably directly recycled) into the isomerization reactor, either separately from the fresh feed into this isomerization reactor or by premixing the isomerate process recycle stream with the fresh feed, wherein the so obtained mixture is fed into the isomerization reactor. In case of two subsequent isomerization reactors, the isomerate process recycle stream is recycled (preferably directly recycled) into the first upstream of the two isomerization reactors, either separately from the fresh feed into this isomerization reactor or by premixing the isomerate process recycle stream with the fresh feed, wherein the so obtained mixture is fed into this isomerization reactor.

The purified n-hexane stream removed from the dividing-wall distillation column in step c) may be further processed. For instance, the purified n-hexane stream removed from the dividing-wall distillation column in step c) may be subjected to a benzene saturation step in a benzene saturation reactor or polishing reactor, respectively. For this purpose, the purified n-hexane stream removed from the dividing-wall distillation column in step c) may be fed together with hydrogen to a mixer for preparing a mixture of the purified n-hexane stream and hydrogen, before the so obtained mixture is optionally preheated and then fed into the benzene saturation reactor, in which at least a part of remaining benzene in the purified n-hexane stream is hydrogenated. The so hydrogenated purified n-hexane stream may then be further processed by subjecting it to a stripping step in a stripper column in order to separate lights (in particular C5- [i.e. C5 minus) hydrocarbons including the hydrogen) from the n-hexane stream. The so obtained purified n-hexane stream preferably has a benzene content of less than 3 ppm wt. and a sulfur content of less than 0.5 ppm wt.

In a second aspect, the present invention relates to a plant, which comprises:
i) an isomerization unit comprising at least one isomerization reactor, wherein the isomerization unit comprises an inlet for a hydrocarbon feed stream and an outlet for an isomerate stream and the at least one isomerization reactor comprises an inlet and an outlet, and
ii) a dividing-wall distillation column, which comprises one inlet and four outlets, wherein the inlet of the dividing-wall distillation column is connected with the outlet for an isomerate stream (preferably stable isomerate stream) of the isomerization unit, wherein a first outlet is for removing from the dividing-wall distillation column a light isomerate stream, a second outlet is for removing from the dividing-wall distillation column a heavy isomerate stream, a third outlet is for removing from the dividing-wall distillation column a purified n-hexane stream and a fourth outlet is for removing from the dividing-wall distillation column an isomerate process recycle stream, wherein the dividing-wall distillation column further comprises a recycle line, which fluidly connects the fourth outlet for removing from the dividing-wall distillation column an isomerate process recycle stream and the inlet of at least one of the at least one isomerization reactor.

The third outlet for removing a purified n-hexane stream from the dividing-wall distillation column is not recycled into the isomerization unit and in particular not into any of the isomerization reactors of the isomerization unit.

The recycle line preferably directly leads into the inlet of at least one of the at least one isomerization reactor.

Alternatively, the recycle line may lead to a mixer, to which also an inlet line to the at least one isomerization reactor leads, for mixing the isomerate process recycle stream and the fresh feed stream of the at least one isomerization reactor, wherein the mixer further comprises an outlet line, which directly leads into the inlet of at least one of the at least one isomerization reactor.

In accordance with a first particular preferred embodiment of the present invention, the dividing-wall distillation column is a top dividing-wall column.

In a first variant of this embodiment, the dividing wall of the top dividing-wall column extends from the upper end of the top dividing-wall column perpendicularly downwards over 20 to 80% and preferably over 20 to 70% of the height of the top dividing-wall column at least essentially vertically downwards. Thus, the top dividing-wall distillation column comprises on one side of the dividing wall a first top section, on the opposite side of the dividing wall a second top section and below the dividing wall a bottom section. Essentially vertically downwards means that the angle between the dividing wall and the length axis of the top dividing-wall distillation column is at most 20°, preferably at most 10°, more preferably at most 5° and most preferably 0°. The top dividing-wall column comprises a first outlet at the overhead of the first top section, a second outlet at the overhead of the second top section, a third outlet at the side of the first top section and a fourth outlet at the bottom of the bottom section, wherein the inlet leads into the first top section of the top dividing-wall column.

The top dividing-wall column preferably comprises an overhead condenser (preferably in an air-cooled heat exchanger) at the first top section of the top dividing-wall distillation column being connected with the outlet at the overhead of the first top section and further with a recycle line into the first top section and an overhead condenser (preferably in an air-cooled heat exchanger) at the second top section of the top dividing-wall distillation column being connected with the outlet at the overhead of the second top section and further with a recycle line into the second top section and/or a bottom reboiler (preferably steam heated) at the bottom section of the top dividing-wall distillation column being connected with the outlet at the bottom of the bottom section and further with a recycle line into the bottom section.

In a second variant of this embodiment, the dividing wall of the top dividing-wall column extends from the upper end of the top dividing-wall column perpendicularly downwards over 5 to 60% and preferably over 10 to 50% of the height of the top dividing-wall column at least essentially vertically downwards, wherein the top dividing-wall column further comprises a partition wall arranged below the dividing wall and comprising an essentially horizontally arranged section and a lower essentially vertically arranged section, wherein the upper essentially horizontally arranged section comprises a first edge and a second edge and the lower essentially vertically arranged section comprises an upper edge and a lower edge. The upper edge of the lower essentially vertically arranged section and the first edge of the upper essentially horizontally arranged section of the partition wall are connected with each other over the whole length of both edges, wherein the second edge of the upper essentially horizontally arranged section of the partition wall is fluid tightly connected with the outer wall of the top dividing-wall column. Thus, the top dividing-wall distillation column comprises on one side of the dividing wall a first top section, on the opposite side of the dividing wall a second top section, in the volume below the essentially horizontally arranged section of the partition wall extending until the lower edge of the essentially vertically arranged section of the partition wall a partitioned section and in the residual volume of the top dividing-wall column a bottom section. The top dividing-wall column of this variant comprises a first outlet at the overhead of the first top section, a second outlet at the overhead of the second top section, a third outlet at the side of the partitioned section and a fourth outlet at the bottom of the bottom section, wherein the inlet leads into the first top section of the top dividing-wall column.

The top dividing-wall column of this variant preferably comprises an overhead condenser (preferably in an air-cooled heat exchanger) at the first top section of the top dividing-wall distillation column being connected with the outlet at the overhead of the first top section and further with a recycle line into the first top section and an overhead condenser (preferably in an air-cooled heat exchanger) at the second top section of the top dividing-wall distillation column being connected with the outlet at the overhead of the second top section and further with a recycle line into the second top section and/or a side condenser (preferably in an air-cooled heat exchanger) at the lower partitioned section of the top dividing-wall distillation column being connected with the outlet at the of the partitioned section and further with a recycle line into the partitioned section and/or a bottom reboiler (preferably steam heated) at the bottom section of the top dividing-wall distillation column being connected with the outlet at the bottom of the bottom section and further with a recycle line into the bottom section.

In accordance with a second particular preferred embodiment of the present invention, the dividing-wall distillation column used in step c) is a bottom dividing-wall column, wherein preferably the dividing wall of the bottom dividing-wall column extends from the lower end of the bottom dividing-wall column perpendicularly upwards over 10 to 60% and preferably over 20 to 50% of the height of the bottom dividing-wall column at least essentially vertically upwards so that the bottom dividing-wall distillation column comprises on one side of the dividing wall a first bottom section, on the opposite side of the dividing wall a second bottom section and above the dividing wall a top section. Again, essentially vertically upwards means that the angle between the dividing wall and the length axis of the bottom dividing-wall distillation column is at most 20°, preferably at most 10°, more preferably at most 5° and most preferably 0°. The bottom dividing-wall column comprises a first outlet at the overhead of the first top section, a second outlet at the side of the second middle section, a third outlet at the side of the second middle section and a fourth outlet at the bottom of the bottom section, wherein the inlet leads into the first top section of the top dividing-wall column.

The bottom dividing-wall column of this embodiment preferably comprises an overhead condenser (preferably in an air-cooled heat exchanger) at the top section of the bottom dividing-wall distillation column being connected with the outlet at the overhead of the top section and further with a recycle line into the top section and/or a bottom reboiler (preferably steam heated) at the first bottom section of the bottom dividing-wall distillation column being connected with the outlet at the bottom of the first bottom section and further with a recycle line into the first bottom section and a bottom reboiler (preferably steam heated) at the bottom of the second bottom section of the bottom dividing-wall distillation column being connected with the outlet at the bottom of the second bottom section and further with a recycle line into the second bottom section.

In accordance with a third particular preferred embodiment of the present invention, the dividing-wall distillation column is a middle dividing-wall column.

In a first variant of this embodiment, the dividing wall of the middle dividing-wall column extends, seen from the bottom to the top of the middle dividing-wall distillation column, from a point being located at 20 to 50% of the distance from the bottom to the top of the middle dividing-wall distillation column to a point being located at 70 to 90% of the distance from the bottom to the top of the middle dividing-wall distillation column at least essentially vertically downwards so that the middle dividing-wall distillation column comprises above the dividing wall a top section, below the dividing wall a bottom section, on one side of the dividing wall a first middle section and on the opposite side of the dividing wall a second middle section. The middle dividing-wall column comprises a first outlet at the overhead of the first top section, a second outlet at the side of the second middle section, a third outlet at the side of the second middle section and a fourth outlet at the bottom of the bottom section, wherein the inlet leads into the first top section of the top dividing-wall column.

The middle dividing-wall column of this variant preferably comprises an overhead condenser (preferably in an air-cooled heat exchanger) at the top section of the middle dividing-wall distillation column being connected with the outlet at the overhead of the top section and further with a recycle line into the top section and/or a bottom reboiler (preferably steam heated) at the bottom section of the middle dividing-wall distillation column being connected with the outlet at the bottom of the bottom section and further with a recycle line into the bottom section.

In a second variant of this embodiment, the dividing wall of the middle dividing-wall column extends, seen from the bottom to the top of the middle dividing-wall distillation column, from a point being located at 40 to 60% of the distance from the bottom to the top of the middle dividing-wall distillation column to a point being located at 70 to 90% of the distance from the bottom to the top of the middle dividing-wall distillation column at least essentially vertically downwards, wherein the middle dividing-wall column further comprises a partition wall arranged below the dividing wall and comprising an essentially horizontally arranged section and a lower essentially vertically arranged section. The upper essentially horizontally arranged section comprises a first edge and a second edge and the lower essentially vertically arranged section comprises an upper edge and a lower edge, wherein the upper edge of the lower essentially vertically arranged section and the first edge of the upper essentially horizontally arranged section of the partition wall are connected with each other over the whole length of both edges. The second edge of the upper essentially horizontally arranged section of the partition wall is fluid tightly connected with the outer wall of the top dividing-wall column, so that the middle dividing-wall distillation column comprises above the dividing wall a top section, on one side of the dividing wall a first middle section, on the opposite side of the dividing wall a second middle section, in the volume below the essentially horizontally arranged section of the partition wall extending until the lower edge of the essentially vertically arranged section of the partition wall a partitioned section and in the residual volume of the middle dividing-wall column a bottom section. The middle dividing-wall column of this variant comprises a first outlet at the overhead of the first top section, a second outlet at the side of the second middle section, a third outlet at the side of the portioned section and a fourth outlet at the bottom of the bottom section, wherein the inlet leads into the first top section of the top dividing-wall column.

The middle dividing-wall column of this variant preferably comprises an overhead condenser (preferably in an air-cooled heat exchanger) at the top section of the middle dividing-wall distillation column being connected with the outlet at the overhead of the top section and further with a recycle line into the top section and/or a side condenser (preferably in an air-cooled heat exchanger) at the partitioned section of the middle dividing-wall distillation column being connected with the outlet at the partitioned section and further with a recycle line into the partitioned section and/or a bottom reboiler (preferably steam heated) at the bottom section of the middle dividing-wall distillation column being connected with the outlet at the bottom of the bottom section and further with a recycle line into the bottom section.

Subsequently, the present invention is described by means of illustrative, but not limiting figures, in which:
- Fig. 1: shows a process flow scheme for the production of n-hexane in accordance with one embodiment of the prior art.
- Fig. 2: shows the process flow scheme of an isomerization unit for the production of only isomerate as the desired product in accordance with the prior art.
- Fig. 3: shows a process flow scheme for the production of n-hexane making use of a deisohexanizer distillation column (DIH), an isomerate recycle column (IRC) and a hexane column in accordance with another embodiment of the prior art.
- Fig. 4: shows the concentration profile inside a conventional DIH column of a process flow scheme shown in fig. 3.
- Fig. 5: shows the concentration profile inside a conventional IRC of a process flow scheme shown in fig. 3.
- Fig. 6: shows a process flow scheme for the production of n-hexane making use of a DIH column in accordance with another embodiment of the prior art.
- Fig. 7: shows a process scheme and plant using a top dividing-wall distillation column in accordance with one embodiment of the present invention.
- Fig. 8: shows a process scheme and plant using a bottom dividing-wall distillation column in accordance with another embodiment of the present invention.
- Fig. 9: shows a process scheme and plant using a middle dividing-wall distillation column in accordance with another embodiment of the present invention.
- Fig. 10: shows a process scheme and plant using a top dividing-wall distillation column further comprising a partition wall in accordance with one embodiment of the present invention.
- Fig. 11: shows a process scheme and plant using a middle dividing-wall distillation column further comprising a partition wall in accordance with another embodiment of the present invention.
- Fig. 12: shows a process scheme and plant showing the isomerization unit in accordance with another embodiment of the present invention.

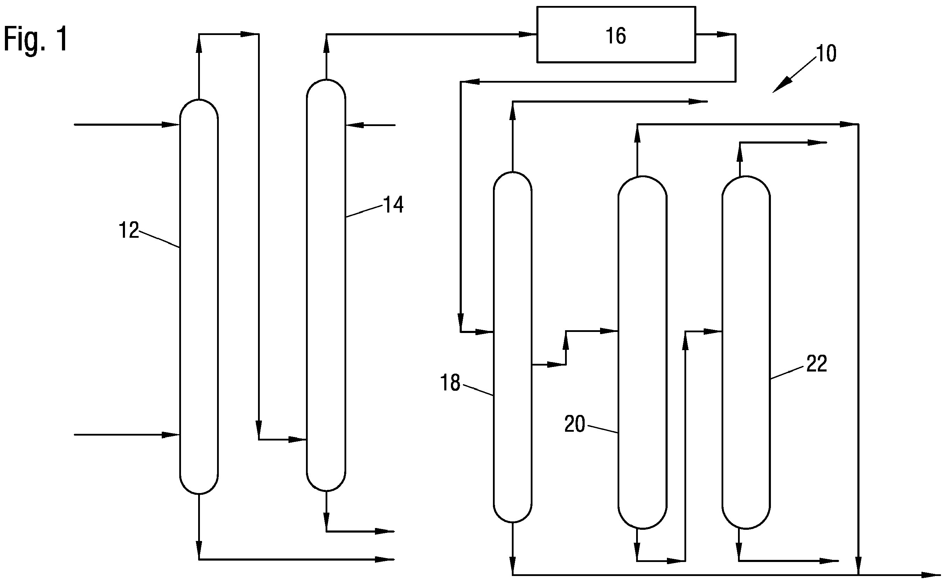

Figure 1 illustrates a conventional prior art solvent extraction process flow scheme 10 for producing hexane and SBP by carrying out extraction of naphtha cut using a solvent. The solvent and naphtha are fed to an extraction column 12, in which the solvent selectively extracts aromatics from the naphtha, thus producing a hydrocarbon stream with a low aromatics content called raffinate. The raffinate from the extraction column 12 is fed to a raffinate wash column 14, in which the raffinate is washed with water in order to remove traces of solvent from the raffinate. The so obtained dearomatized naphtha is then treated in a mercaptan removal unit 16 in order to remove sulfur compounds to thereby produce a dearomatized naphtha stream meeting the sulfur specification. The dearomatized naphtha is then fractionated in a series of three splitter columns 18, 20, 22 so as to produce the desired hexane and SBP cuts. Even if such a solvent extraction process produces n-hexane, its quality is inferior. For example, the benzene and sulfur contents of the n-hexane produced by the solvent extraction process of Figure 1 are high, with the benzene content being about up to 500 ppm wt. and the sulfur content being about 5 ppm wt., respectively.

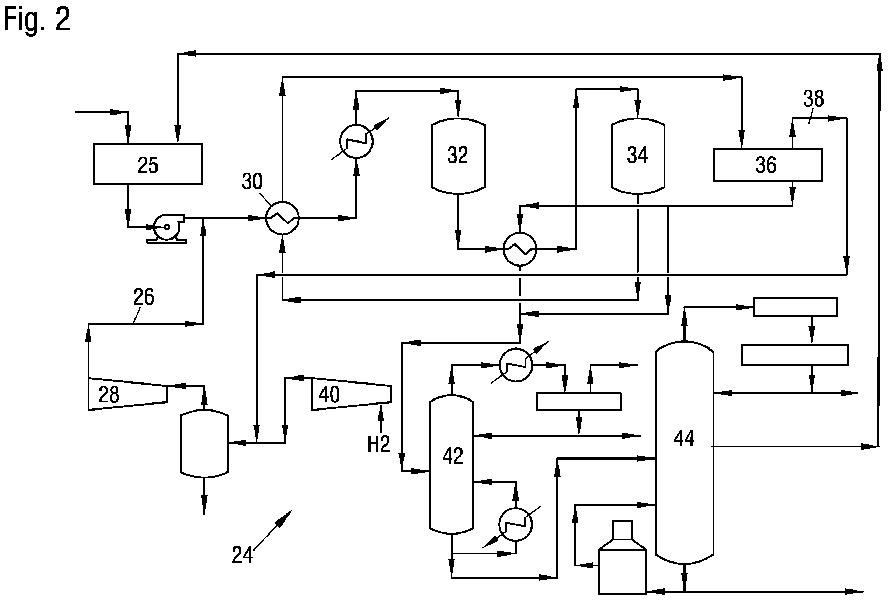

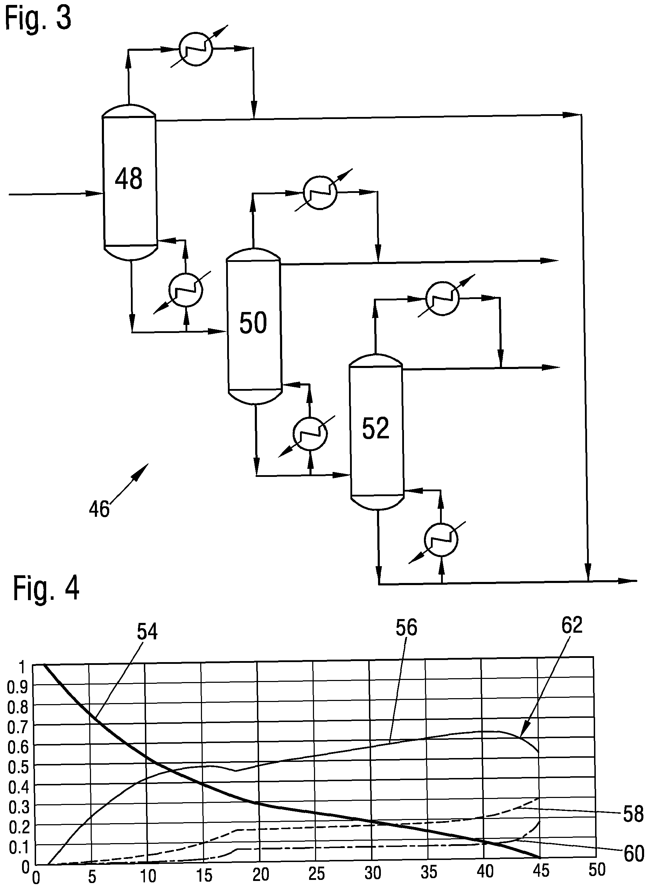

Figure 2 illustrates a conventional prior art isomerization process flow scheme 24. As shown in Figure 2, feed 25 and recycle gas 26 from a recycle gas compressor (RGC) 28, are preheated in a reactor feed-effluent exchanger 30 to a desired temperature before being routed to a series of reactors, for example a reactor one 32 and a reactor two (34), in which saturation of aromatics and conversion of normal paraffins to iso-paraffins takes place. Gas and liquid in the reactor effluent are separated in a product separator 36. Gas 38 from the product separator 36 is recycled back, using the RGC 28, to a reaction section after adding makeup hydrogen by using a makeup gas (MUG) compressor 40. Liquid, from the product separator 36, is routed to a stabilizer 42 for stabilization by removal of gas and liquefied petroleum gas (LPG) from the liquid. The stabilized isomerate is then split in a deisohexanizer (DIH) distillation column 44 to produce isomerate, meeting the specification of octane etc. Isomerate, thus produced, is a blend component of the refinery gasoline pool. The isomerization process flow scheme 24 produces only isomerate as the desired product. This isomerization process produces only isomerate as the desired product. However, instead operating the DIH distillation column as described above, it is also known to produce a purified n-hexane stream from the stable isomerate produced as described below with regard to figure 3, before it is fed into the DIH column 44.

Figure 3 shows a prior art process flow scheme 46 for producing a purified n-hexane stream from a stable isomerate stream, such as a stable isomerate stream produced in a plant in accordance with figure 2, making use of a deisohexanizer distillation column 48, an isomerate recycle distillation column 50 and a n-hexane distillation column 52 in accordance with another embodiment of the prior art. The bottom fraction obtained in the deisohexanizer distillation column 48 is fed to the isomerate recycle distillation column 50 and the bottom fraction obtained in the isomerate recycle distillation column 50 is fed into the n-hexane column 52. The deisohexanizer distillation column 48 produces light isomerate as overhead fraction and the isomerate recycle distillation column 50 splits the isomerization process recycle as overhead fraction or overhead cut, respectively. The n-hexane column 52 produces high-purity hexane and heavy isomerate as top and bottom fractions or cuts, respectively. The prior art plant 46 of Figure 3 has certain disadvantages, namely that the boiling points of C6 hydrocarbons are very close to that of the non-desirable components, such as e.g. C6 n-alkans, single branched alkanes and cycloalkanes. In order to obtain a good separation between the light isomerate and the isomerate process recycle stream along with high purity hexane meeting specifications for its narrow boiling range and C6 cycloalkanes, the process of the plant 46 requires a significant number of trays, leading to bigger columns as well as high re-boiling energy. Such plants with three distillation columns also have the problem of back-mixing of a concentrated light isomerate, which is rich in double branched C6 iso-alkanes, and the isomerate process recycle stream, which is rich in single branched C6 i-Paraffins, in DIH Column as well as back-mixing of high purity hexane, which is rich in C6 iso-alkanes, and the heavy isomerate stream, which is rich in C6 cycloalkanes, within the isomerate recycle distillation column 50. Thus, the energy spent in concentrating these streams to higher purity levels is lost due to the back-mixing of different C6 iso-alkanes, n-hexane with C6 cycloalkanes and heavy isomerate at the bottom of the isomerate recycle distillation column 50.

The concentration profiles of the light isomerate 54, isomerate process recycle stream 56, high purity n-hexane 58 and heavy isomerate 60 fractions in the deisohexanizer distillation column 48 are shown in figure 4. The ordinate of the graph shows the mol fraction and the abscissa shows the number of theoretical stages. The arrow at position 64 shows the thermodynamic inefficiency, namely that the recycle mixes back into hexane and heavy isomerate.

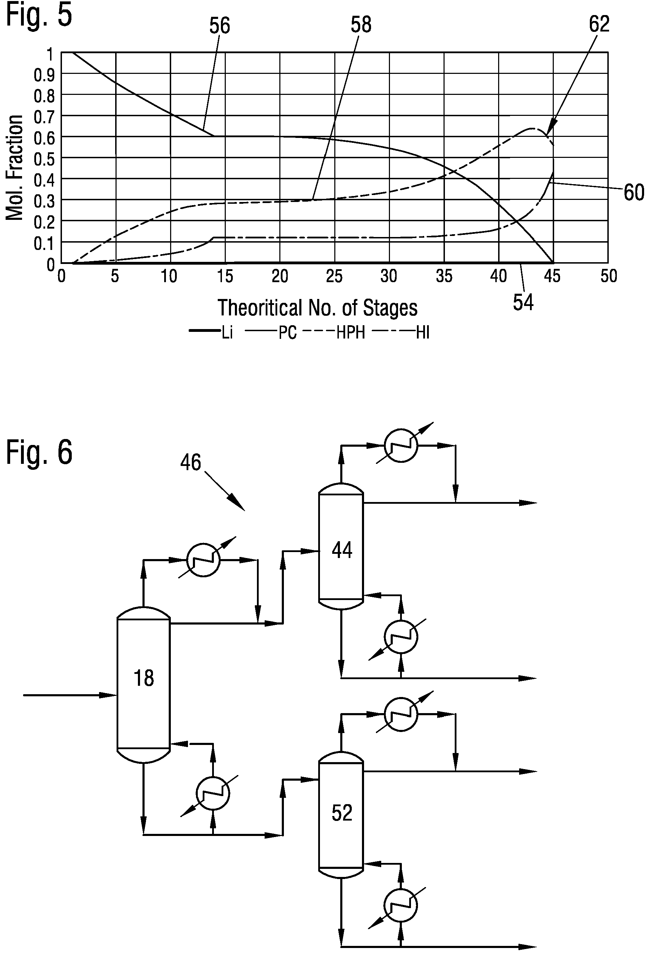

Additional energy is spent in the isomerate recycle distillation column 50, as shown in figure 5 being configured as figure 4, to separate the isomerate process recycle stream and the high purity n-hexane stream from heavy isomerate, thereby reducing an overall energy efficiency of the process.

Figure 6 shows a prior art process flow scheme for the production of n-hexane making use of a DIH column 44 in accordance with another embodiment of the prior art. The plant 46 shown in figure 6 comprises three distillation columns 18, 44, 52 arranged such that overhead and bottom fractions from first (splitter) distillation column 18 are further split in two parallel distillation columns 44, 52, namely in a deisohexanizer column 44 and in a n-hexane column 52. Figure 6 illustrates a plant for splitting stable isomerate into a light isomerate stream, into an isomerization process recycle stream, into a high-purity n-hexane stream and into a heavy isomerate stream. More specifically, the plant 46 includes a splitter distillation column 18, a deisohexanizer distillation column 44 and a n-hexane distillation column 52. The overhead fraction obtained in the deisohexanizer distillation column 44 is fed to the deisohexanizer column 44 and the bottom fraction obtained in the splitter distillation column 18 is fed into the n-hexane distillation column 52. The deisohexanizer distillation column 44 produces a light isomerate stream as overhead fraction and an isomerate process recycle stream as bottom fraction. The n-hexane column 52 produces a high purity n-hexane stream as overhead fraction and a heavy isomerate stream as bottom fraction. The prior art process performed in the plant 46 shown in figure 6 also has similar disadvantages as that performed in the plant 46 shown in figure 3.

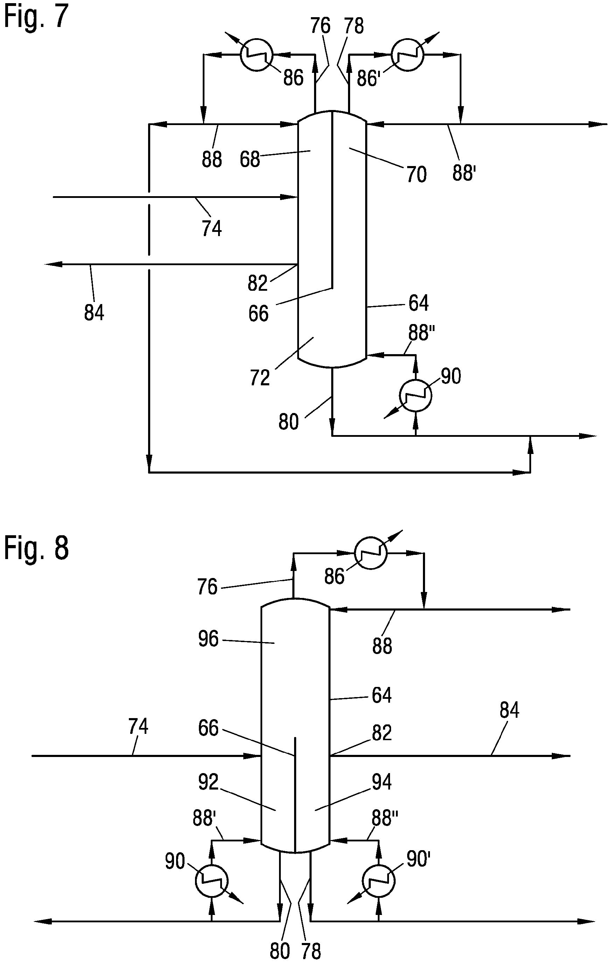

Figure 7 shows a dividing-wall distillation column 64 to be used in accordance with the present invention. The dividing-wall distillation column 64 shown in figure 7 is a top dividing wall column 64 comprising a dividing wall 66, which extends from the upper end of the dividing-wall column 64 over about 60% of the height of the dividing-wall column 64 vertically downwards. Therefore, the dividing-wall distillation column 64 comprises on one side of the dividing wall 66 a first top section 68, on the opposite side of the dividing wall 66 a second top section 70 and below the dividing wall 66 a bottom section 72. The dividing-wall distillation column 64 comprises one inlet 74 and four outlets 76, 78, 80, 82. The inlet 74 of the dividing-wall distillation column enters the dividing-wall column 64 at the side of the first top section 68 and is connected with the outlet for an isomerate stream of the isomerization unit (not shown in figure 7). The first outlet 76 is for removing from the dividing-wall distillation column 64 a light isomerate stream and exits the dividing-wall distillation column 64 at the head of the first top section 68, whereas the second outlet 78 is for removing from the dividing-wall distillation column 64 a purified n-hexane stream and exits the dividing-wall distillation column 64 at the head of the second top section 70. The third outlet 80 is for removing from the dividing-wall distillation column 64 a heavy isomerate stream and exits the dividing-wall distillation column 64 at the bottom of the bottom section 72 and the fourth outlet 82 is for removing from the dividing-wall distillation column 64 an isomerate process recycle stream and exits the dividing-wall distillation column 64 at the side of the first top section 68. The dividing-wall distillation column 64 further comprises a recycle line 84, which fluidly connects the fourth outlet 82 for removing from the dividing-wall distillation column 64 the isomerate process recycle stream and the inlet of at least one of the at least one isomerization reactor (not shown in figure 7). The dividing-wall column 64 further comprises an overhead condenser 86, preferably in an air-cooled heat exchanger, at the first top section 68 of the dividing-wall distillation column 64, which is connected with the outlet 76 at the overhead of the first top section 68 and further with a recycle line 88 into the first top section 68. Moreover, the dividing-wall distillation column 64 further comprises an overhead condenser 86', preferably in an air-cooled heat exchanger, at the second top section 70 of the top dividing-wall distillation column 64, which is connected with the outlet at the overhead of the second top section 70 and further with a recycle line 88' into the second top section 70. In addition, the dividing-wall distillation column 64 further comprises a bottom reboiler 90, which is preferably steam heated, at the bottom section 72 of the dividing-wall distillation column 64, which is connected with the outlet 80 at the bottom of the bottom section 72 and further with a recycle line 88" into the bottom section 72. During the operation, stable isomerate stream is fed as side fraction into the first top section 68 of the dividing-wall distillation column 64, light isomerate stream is removed as overhead fraction from the first top section 68 of the dividing-wall distillation column 64 though outlet 76, purified n-hexane stream is removed as overhead fraction from the second top section 70 of the dividing-wall distillation column 64 though outlet 78, heavy isomerate stream is removed as bottom fraction from the bottom section 72 of the dividing-wall distillation column 64 through outlet 80 and the isomerate process recycle stream is removed as side fraction from the first top section 68 of the top dividing-wall distillation column 64 through outlet 82.

Figure 8 shows a dividing-wall distillation column 64 according to another embodiment to be used in accordance with the present invention. The dividing-wall distillation column 64 shown in figure 8 is a bottom dividing wall column 64 comprising a dividing wall 66, which extends from the bottom end of the dividing-wall column 64 over about 40% of the height of the dividing-wall column 64 vertically upwards. Therefore, the dividing-wall distillation column 64 comprises on one side of the dividing wall 66 a first bottom section 92, on the opposite side of the dividing wall 66 a second bottom section 94 and above the dividing wall 66 a top section 96. The dividing-wall distillation column 64 comprises one inlet 74 and four outlets 76, 78, 80, 82. The inlet 74 of the dividing-wall distillation column enters the dividing-wall column 64 at the side of the first bottom section 92 and is connected with the outlet for an isomerate stream of the isomerization unit (not shown in figure 8). The first outlet 76 is for removing from the dividing-wall distillation column 64 a light isomerate stream and exits the dividing-wall distillation column 64 at the head of the top section 96, whereas the second outlet 78 is for removing from the dividing-wall distillation column 64 a purified n-hexane stream and exits the dividing-wall distillation column 64 at the bottom of the second bottom section 94. The third outlet 80 is for removing from the dividing-wall distillation column 64 a heavy isomerate stream and exits the dividing-wall distillation column 64 at the bottom of the first bottom section 92 and the fourth outlet 82 is for removing from the dividing-wall distillation column 64 an isomerate process recycle stream and exits the dividing-wall distillation column 64 at the side of the second bottom section 94. The dividing-wall distillation column 64 further comprises a recycle line 84, which fluidly connects the fourth outlet 82 for removing from the dividing-wall distillation column 64 the isomerate process recycle stream and the inlet of at least one of the at least one isomerization reactor (not shown in figure 8). The dividing-wall column 64 further comprises an overhead condenser 86, preferably in an air-cooled heat exchanger, at the top section 96 of the dividing-wall distillation column 64, which is connected with the outlet 76 at the overhead of the top section 96 and further with a recycle line 88 into the top section 96. Moreover, the dividing-wall distillation column 64 further comprises a first bottom reboiler 90, which is preferably steam heated, at the first bottom section 92 of the dividing-wall distillation column 64, which is connected with the outlet 80 at the bottom of the first bottom section 92 and further with a recycle line 88' into the first bottom section 92. In addition, the dividing-wall distillation column 64 further comprises a second bottom reboiler 90', which is preferably steam heated, at the second bottom section 94 of the dividing-wall distillation column 64, which is connected with the outlet 78 at the bottom of the second bottom section 94 and further with a recycle line 88" into the second bottom section 94.

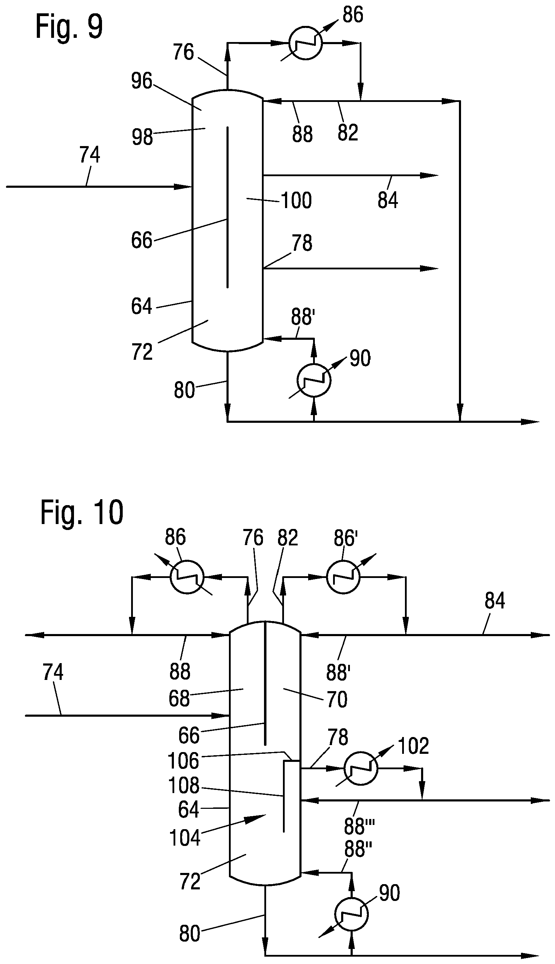

Figure 9 shows a dividing-wall distillation column 64 according to another embodiment to be used in accordance with the present invention. The dividing-wall distillation column 64 shown in figure 9 is a middle dividing wall column 64 comprising a dividing wall 66, which extends, seen from the bottom to the top of the dividing-wall distillation column 64, from a point being located about 25% of the distance from the bottom to the top of the dividing-wall distillation column 64 to a point being located at about 90% of the distance from the bottom to the top of the dividing-wall distillation column 64 vertically upwards. Therefore, the dividing-wall distillation column 64 comprises on one side of the dividing wall 66 a first side section 98, on the opposite side of the dividing wall 66 a second side section 100, above the dividing wall 66 a top section 96 and below the dividing wall 66 a bottom section 72. The dividing-wall distillation column 64 comprises one inlet 74 and four outlets 76, 78, 80, 82. The inlet 74 of the dividing-wall distillation column enters the dividing-wall column 64 at the side of the first side section 98 and is connected with the outlet for an isomerate stream of the isomerization unit (not shown in figure 9). The first outlet 76 is for removing from the dividing-wall distillation column 64 a light isomerate stream and exits the dividing-wall distillation column 64 at the head of the top section 96, whereas the second outlet 78 is for removing from the dividing-wall distillation column 64 a purified n-hexane stream and exits the dividing-wall distillation column 64 at the side of the second side section 100. The third outlet 80 is for removing from the dividing-wall distillation column 64 a heavy isomerate stream and exits the dividing-wall distillation column 64 at the bottom of the bottom section 72 and the fourth outlet 82 is for removing from the dividing-wall distillation column 64 an isomerate process recycle stream and exits the dividing-wall distillation column 64 at the side of the second side section 100 above the outlet 78. The dividing-wall distillation column 64 further comprises a recycle line 84, which fluidly connects the fourth outlet 82 for removing from the dividing-wall distillation column 64 the isomerate process recycle stream and the inlet of at least one of the at least one isomerization reactor (not shown in figure 9). The dividing-wall column 64 further comprises an overhead condenser 86, preferably in an air-cooled heat exchanger, at the top section 96 of the dividing-wall distillation column 64, which is connected with the outlet 76 at the overhead of the top section 96 and further with a recycle line 88 into the top section 96. Moreover, the dividing-wall distillation column 64 further comprises a bottom reboiler 90, which is preferably steam heated, at the bottom section 72 of the dividing-wall distillation column 64, which is connected with the outlet 80 at the bottom of the bottom section 72 and further with a recycle line 88' into the bottom section 72.

Figure 10 shows a dividing-wall distillation column 64 according to another embodiment to be used in accordance with the present invention. The dividing-wall distillation column 64 shown in figure 10 is a top dividing wall column 64 being similar to that shown in figure 7. However, the dividing wall column 64 of the embodiment of figure 10 further comprises a lower partition wall 104, which is arranged below the dividing wall 66. The partition wall 104 comprises a horizontally arranged section 106 and a lower vertically arranged section 108, wherein the upper horizontally arranged section 106 comprises a first edge and a second edge and the lower essentially vertically arranged section comprises an upper edge and a lower edge. The upper edge of the lower vertically arranged section 108 and the first edge of the upper horizontally arranged section 106 of the partition wall 104 are connected with each other over the whole length of both edges, wherein the second edge of the upper horizontally arranged section 106 of the partition wall 104 is fluid tightly connected with the outer wall of the top dividing-wall column 64. In further difference to the dividing-wall distillation column 64 shown in figure 7, the wall distillation column 64 shown in figure 10 has the outlet has the outlet for the isomerate process recycle stream located at the overhead of the second top section 70 of the dividing-wall distillation column 64, whereas the outlet 78 for the purified n-hexane stream is located at the partitioned section of the distillation column 64, which is, as set out further above, the volume below the horizontally arranged section 106 of the partition wall 104 extending until the lower edge of the vertically arranged section 108 of the partition wall 104. The dividing-wall column 64 further comprises a side condenser 102, preferably in an air-cooled heat exchanger, which is connected with the outlet 78 and further with a recycle line 88‴ into the partitioned section of the distillation column 64.

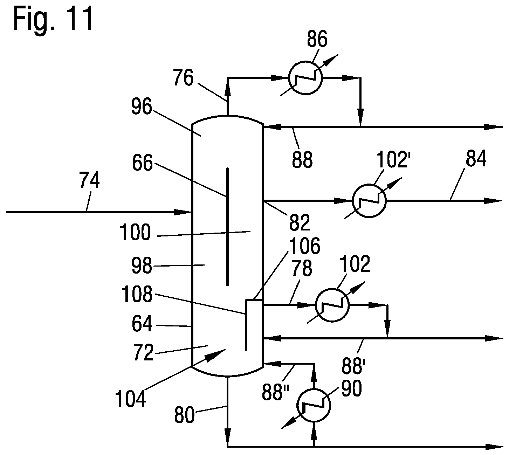

Figure 11 shows a dividing-wall distillation column 64 according to another embodiment to be used in accordance with the present invention. The dividing-wall distillation column 64 shown in figure 11 is a middle dividing wall column 64 being similar to that shown in figure 9. However, the dividing wall column 64 of the embodiment of figure 10 further comprises a lower partition wall 104, which is arranged below the dividing wall 66. The partition wall 104 is embodied so as described above for figure 11. Moreover, the dividing wall 66 of the dividing-wall distillation column 64 of figure 11 is shorter than the dividing wall 66 of the dividing-wall distillation column 64 of figure 9. In addition, the outlet 78 is provided with a side condenser 102 and a recycle line 88'. Also, the outlet 82 is provided with a side condenser 102'.

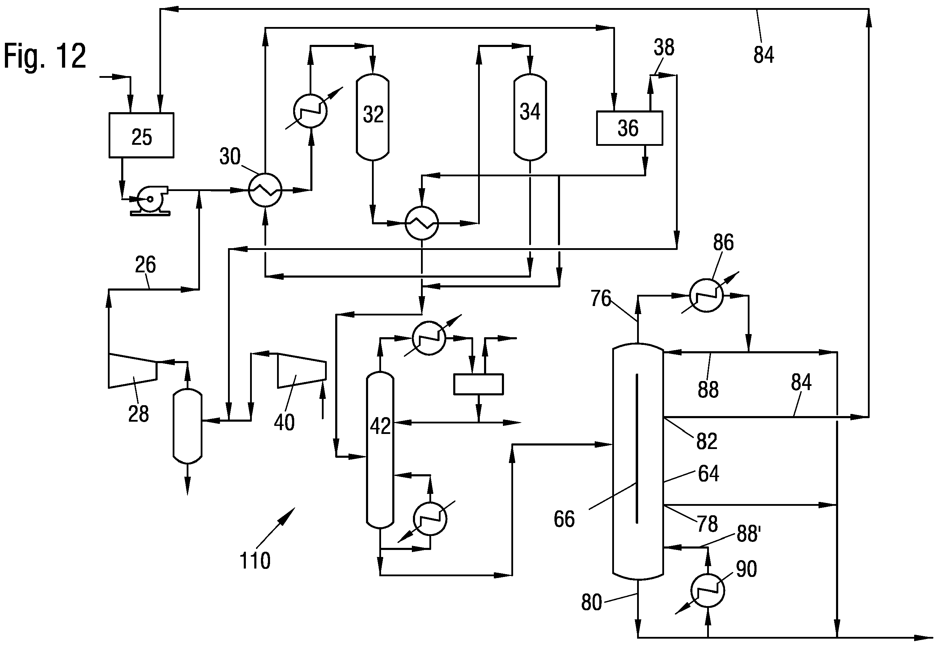

Figure 12 shows a plant showing of an isomerization unit 110, which comprises as dividing wall column 64 a middle dividing wall column 64 of the embodiment shown in figure 9. The recycle line 84 for the isomerate process recycle stream of the dividing wall column 64 is connected with the feed 25. The other parts of the isomerization unit 100 of figure 12 are as described for figure 2.

Subsequently, the present invention is described by means of illustrative, but not limiting examples.

### Examples

Tables 1 to 8 below demonstrate various operating parameters for conventional processes and systems and processes and systems of the present patent application that utilizes 4-cut divided-wall distillation columns with an isomerate process recycle stream.

### Table 1a &1b : Performance of Prior Art plant versus the plant in accordance with the present invention

**Table 1a:Prior Art**

| **Items** | **Units** | | **Conventional Series** | | | **Conventional Parallel** | | |
|---|---|---|---|---|---|---|---|---|
| Columns | | **DIH with no HPH** | **DIH** | **Isom Recycle** | **Hexane** | **Splitter** | **DIH** | **Hexane** |
| | | **Fig 2** | **Fig. 3** | | | **Fig. 6** | | |
| Feed | kg/hr | 40000 | 40,000 | | | 40,000 | | |
| Light Isomerate | kg/hr | 17051 | 17,800 | | | 17,800 | | |
| Heavy Isomerate | kg/hr | 2986 | 4,000 | | | 4,001 | | |
| Isomerate Recycle | kg/hr | 19963 | 11,850 | | | 11,848 | | |
| **High-purity Hexane (HPH)** | kg/hr | **0** | **6,350** | | | **6,351** | | |
| n-Hexane content | wt% | - | 40.1 | | | 40.56 | | |
| Reboiler Duty | MMkcal/hr | 6.1 | 8.6 | 5.3 | 2.6 | 8.4 | 5.8 | 2.7 |
| Total Reboiler Duty | MMkcal/hr | **6.1** | 16.5 | | | 16.9 | | |
| Condenser Duty | MMkcal/hr | 8.25 | 7.8 | 5.1 | 2.7 | 6.4 | 5.9 | 2.7 |
| Additional Duty for HPH | MMkcal/hr | - | 10.4 | | | 10.8 | | |
| Duty/ton of HPH | MMkcal/ton | - | 1.64 | | | 1.70 | | |
| **Energy Savings/ton of HPH produced** | **%** | | **3.7** | | | **Base** | | |

| | | | | | | | | |
|---|---|---|---|---|---|---|---|---|
| HPH - High-purity hexane DIH - Deisohexanizer | | | | | | | | |

**Table 1b: According to the present invention**

| **Items** | **Units** | **Four cut dividing wall column** | | | | |
|---|---|---|---|---|---|---|
| **Columns** | | **TDW Column with side stream** | **BDW Column with side stream** | **MDW Column with side stream** | **TDW & PW Column** | **MDW & PW Column** |
| | | **Fig. 7** | **Fig. 8** | **Fig. 9** | **Fig. 10** | **Fig. 11** |
| Feed | kg/hr | 40000 | 40000 | 40000 | 40000 | 40000 |
| Light Isomerate | kg/hr | 17850 | 17750 | 17850 | 17800 | 17800 |
| Heavy Isomerate | kg/hr | 3150 | 4200 | 3950 | 4050 | 4000 |
| High-purity Hexane(HPH) | kg/hr | **4050** | **3650** | **6450** | **6350** | **6450** |
| n-Hexane content | wt% | 40.99 | 40.41 | 40.85 | 40.05 | 40.01 |
| Total Reboiler Duty | MMkcal/hr | 10.6 | 11.80 | 10.60 | 11.80 | 10.60 |
| Condenser Duty | MMkcal/hr | 12.7 | 14.2 | 12.6 | 14.2 | 13.3 |
| Additional Duty for HPH | MMkcal/hr | 4.5 | 5.7 | 4.5 | 5.7 | 4.5 |
| Duty/ton of HPH | MMkcal/ton | 1.1 | 1.6 | 0.7 | 0.9 | 0.7 |
| **Energy Savings/ton of HPH** | **%** | **34.7** | **8.2** | **59.0** | **47.2** | **59.0** |

| | | | | | | |
|---|---|---|---|---|---|---|
| TDW - Top divising-wall column BDW - Bottom divising-wall column MDW - Middle divising-wall column PW - Partition wall | | | | | | |

**Table 2a**

| **Material balance of Prior Art DIH column + Isom Recycle + Hexane column in series of** **Figure 3** | | | | | | |
|---|---|---|---|---|---|---|
| **Stream Description** | **Units** | **Feed** | **Light** | **Isom recycle** | **High-purity** | **Heavy** |
| | | | **Isomerate** | | **Hexane** | **Isomerate** |
| Flowrate | kg/hr | 40000.00 | 17799.56 | 11850.05 | 6349.92 | 4000.48 |
| Composition profile | | | | | | |
| H2 | wt. % | 0 | 0 | 0 | 0 | 0 |
| C3- | wt. % | 0 | 0 | 0 | 0 | 0 |
| C4 Paraffins | wt. % | 0.00 | 0.00 | 0.00 | 0.00 | 0.00 |
| i-Pentane | wt. % | 13.41 | 30.14 | 0.00 | 0.00 | 0.00 |
| n-Pentane | wt. % | 3.90 | 8.77 | 0.00 | 0.00 | 0.00 |
| C5 Naphthenes | wt. % | 0.50 | 1.12 | 0.01 | 0.00 | 0.00 |
| C6 i-Paraffins (2,2 & 2,3MB) | wt. % | 29.53 | **51.45** | 21.49 | 1.69 | 0.00 |
| C6 i-Paraffins (2 & 3 MP) | wt. % | 32.03 | 8.42 | **72.24** | 43.32 | 0.09 |
| Hexane | wt. % | 8.01 | 0.08 | 5.12 | 40.09 | 0.92 |
| C6 Naphthenes | wt. % | 5.81 | 0.02 | 1.14 | 14.91 | 30.93 |
| Benzene | wt. % | 0 | 0 | 0 | 0 | 0 |
| C7 Paraffins | wt. % | 3.10 | 0.00 | 0.00 | 0.00 | 31.03 |
| C7 Naphthenes | wt. % | 3.70 | 0.00 | 0.00 | 0.00 | 37.03 |
| Total | wt. % | 100 | 100 | 100 | 100 | 100 |
| Reboiler Duty | MMkcal/hr | 14.53 | | | | |
| Condenser Duty | MMkcal/hr | 16.48 | | | | |

**Table 2b**

| **Material balance of conventional Splitter column + Light Isomerate + Hexane column in series of** **Figure 6** | | | | | | |
|---|---|---|---|---|---|---|
| **Stream Description** | **Units** | **Feed** | **Light** | **Isom recycle** | **High-purity** | **Heavy** |
| | | | **Isomerate** | | **Hexane** | **Isomerate** |
| Flowrate | kg/hr | 40000.00 | 17799.99 | 11847.95 | 6350.74 | 4001.32 |
| Composition profile | | | | | | |
| H2 | wt. % | 0 | 0 | 0 | 0 | 0 |
| C3- | wt. % | 0 | 0 | 0 | 0 | 0 |
| C4 Paraffins | wt. % | 0.00 | 0.00 | 0.00 | 0.00 | 0.00 |
| i-Pentane | wt. % | 13.41 | 30.14 | 0.00 | 0.00 | 0.00 |
| n-Pentane | wt. % | 3.90 | 8.77 | 0.00 | 0.00 | 0.00 |
| C5 Naphthenes | wt. % | 0.50 | 1.12 | 0.01 | 0.00 | 0.00 |
| C6 i-Paraffins (2,2 &2,3MB) | wt. % | 29.53 | **51.45** | 20.71 | 3.15 | 0.00 |
| C6 i-Paraffins (2 & 3 MP) | wt. % | 32.03 | 8.49 | **73.30** | 41.20 | 0.03 |
| Hexane | wt. % | 8.01 | 0.02 | 5.09 | 40.56 | 0.50 |
| C6 Naphthenes | wt. % | 5.81 | 0.00 | 0.89 | 15.09 | 31.43 |
| Benzene | wt. % | 0 | 0 | 0 | 0 | 0 |
| C7 Paraffins | wt. % | 3.10 | 0.00 | 0.00 | 0.00 | 31.02 |
| C7 Naphthenes | wt. % | 3.70 | 0.00 | 0.00 | 0.00 | 37.02 |
| Total | wt. % | 100 | 100 | 100 | 100 | 100 |
| Reboiler Duty | MMkcal/hr | 14.99 | | | | |
| Condenser Duty | MMkcal/hr | 16.85 | | | | |

**Table 3**

| **Material balance of Top Dividing wall (TDW) column with side stream of Figure 7** | | | | | | |
|---|---|---|---|---|---|---|
| **Stream Description** | **Units** | **Feed** | **Light** | **Isom recycle** | **High-purity** | **Heavy** |
| | | | **Isomerate** | | **Hexane** | **Isomerate** |
| Flowrate | kg/hr | 40000.00 | 17850.00 | 14950.00 | 4050.00 | 3150.00 |
| Composition profile | | | | | | |
| H2 | wt. % | 0 | 0 | 0 | 0 | 0 |
| C3- | wt. % | 0 | 0 | 0 | 0 | 0 |
| C4 Paraffins | wt. % | 0.00 | 0.00 | 0.00 | 0.00 | 0.00 |
| i-Pentane | wt. % | 13.41 | 30.06 | 0.00 | 0.00 | 0.00 |
| n-Pentane | wt. % | 3.90 | 8.75 | 0.00 | 0.00 | 0.00 |
| C5 Naphthenes | wt. % | 0.50 | 1.11 | 0.01 | 0.00 | 0.00 |
| C6 i-Paraffins (2,2 &2,3 MB) | wt. % | 29.53 | **51.45** | 16.87 | 2.62 | 0.00 |
| C6 i-Paraffins (2 & 3 MP) | wt. % | 32.03 | 8.55 | **64.39** | 41.00 | 0.00 |
| Hexane | wt. % | 8.01 | 0.08 | 10.22 | 40.99 | 0.01 |
| C6 Naphthenes | wt. % | 5.81 | 0.01 | 5.30 | 15.39 | 28.79 |
| Benzene | wt. % | 0 | 0 | 0 | 0 | 0 |
| C7 Paraffins | wt. % | 3.10 | 0.00 | 1.52 | 0.00 | 32.20 |
| C7 Naphthenes | wt. % | 3.70 | 0.00 | 1.69 | 0.00 | 39.01 |
| Total | wt. % | 100 | 100 | 100 | 100 | 100 |
| Reboiler Duty | MMkcal/hr | 10.6 | | | | |
| Condenser Duty | MMkcal/hr | 12.66 | | | | |

**Table 4**

| **Material balance of Bottom Dividing wall (BDW) column with side stream of** **Figure 8** | | | | | | |
|---|---|---|---|---|---|---|
| **Stream Description** | **Units** | **Feed** | **Light** | **Isom recycle** | **High-purity** | **Heavy** |
| | | | **Isomerate** | | **Hexane** | **Isomerate** |
| Flowrate | kg/hr | 40000.00 | 17750.00 | 14400.00 | 3650.00 | 4200.00 |
| Composition profile | | | | | | |
| H2 | wt. % | 0 | 0 | 0 | 0 | 0 |
| C3- | wt. % | 0 | 0 | 0 | 0 | 0 |
| C4 Paraffins | wt. % | 0.00 | 0.00 | 0.00 | 0.00 | 0.00 |
| i-Pentane | wt. % | 13.41 | 30.21 | 0.02 | 0.00 | 0.00 |
| n-Pentane | wt. % | 3.90 | 8.77 | 0.03 | 0.00 | 0.00 |
| C5 Naphthenes | wt. % | 0.50 | 1.09 | 0.05 | 0.00 | 0.00 |
| C6 i-Paraffins (2,2 &2,3 MB) | wt. % | 29.53 | **51.28** | 18.35 | 1.66 | 0.01 |
| C6 i-Paraffins (2 & 3 MP) | wt. % | 32.03 | 8.65 | **67.03** | 43.96 | 0.21 |
| Hexane | wt. % | 8.01 | 0.00 | 11.41 | 40.41 | 2.20 |
| C6 Naphthenes | wt. % | 5.81 | 0.00 | 3.12 | 13.97 | 32.75 |
| Benzene | wt. % | 0 | 0 | 0 | 0 | 0 |
| C7 Paraffins | wt. % | 3.10 | 0.00 | 0.00 | 0.00 | 29.55 |
| C7 Naphthenes | wt. % | 3.70 | 0.00 | 0.00 | 0.00 | 35.27 |
| Total | wt. % | 100 | 100 | 100 | 100 | 100 |
| Reboiler Duty | MMkcal/hr | 11.8 | | | | |
| Condenser Duty | MMkcal/hr | 14.15 | | | | |

**Table 5**

| **Material balance of Middle Dividing wall (MDW) column with side stream of** **Figure 9** | | | | | | |
|---|---|---|---|---|---|---|
| **Stream Description** | **Units** | **Feed** | **Light** | **Isom recycle** | **High-purity** | **Heavy** |
| | | | **Isomerate** | | **Hexane** | **Isomerate** |
| Flowrate | kg/hr | 40000.00 | 17849.99 | 11750.11 | 6450.29 | 3949.57 |
| Composition profile | | | | | | |
| H2 | wt. % | 0 | 0 | 0 | 0 | 0 |
| C3- | wt. % | 0 | 0 | 0 | 0 | 0 |
| C4 Paraffins | wt. % | 0.00 | 0.00 | 0.00 | 0.00 | 0.00 |
| i-Pentane | wt. % | 13.41 | 30.03 | 0.05 | 0.00 | 0.00 |
| n-Pentane | wt. % | 3.90 | 8.71 | 0.06 | 0.00 | 0.00 |
| C5 Naphthenes | wt. % | 0.50 | 1.08 | 0.06 | 0.00 | 0.00 |
| C6 i-Paraffins (2,2 &2,3 MB) | wt. % | 29.53 | **50.67** | 22.53 | 2.18 | 0.00 |
| C6 i-Paraffins (2 & 3 MP) | wt. % | 32.03 | 9.49 | **72.01** | 43.14 | 0.02 |
| Hexane | wt. % | 8.01 | 0.02 | 4.38 | 40.85 | 0.43 |
| C6 Naphthenes | wt. % | 5.81 | 0.00 | 0.91 | 13.83 | 30.67 |
| Benzene | wt. % | 0 | 0 | 0 | 0 | 0 |
| C7 Paraffins | wt. % | 3.10 | 0.00 | 0.00 | 0.00 | 31.39 |
| C7 Naphthenes | wt. % | 3.70 | 0.00 | 0.00 | 0.00 | 37.49 |
| Total | wt. % | 100 | 100 | 100 | 100 | 100 |
| Reboiler Duty | MMkcal/hr | 10.6 | | | | |
| Condenser Duty | MMkcal/hr | 12.58 | | | | |

**Table 6**

| **Material balance of TDW & partition wall (PW) column of** **Figure 10** | | | | | | |
|---|---|---|---|---|---|---|
| **Stream Description** | **Units** | **Feed** | **Light** | **Isom recycle** | **High-purity** | **Heavy** |
| | | | **Isomerate** | | **Hexane** | **Isomerate** |
| Flowrate | kg/hr | 40000.00 | 17800.00 | 11799.99 | 6349.92 | 4050.22 |
| Composition profile | | | | | | |
| H2 | wt. % | 0 | 0 | 0 | 0 | 0 |
| C3- | wt. % | 0 | 0 | 0 | 0 | 0 |
| C4 Paraffins | wt. % | 0.00 | 0.00 | 0.00 | 0.00 | 0.00 |
| i-Pentane | wt. % | 13.41 | 30.14 | 0.00 | 0.00 | 0.00 |
| n-Pentane | wt. % | 3.90 | 8.77 | 0.00 | 0.00 | 0.00 |
| C5 Naphthenes | wt. % | 0.50 | 1.12 | 0.01 | 0.00 | 0.00 |
| C6 i-Paraffins (2,2 &2,3 MB) | wt. % | 29.53 | **51.16** | 21.31 | 3.00 | 0.00 |
| C6 i-Paraffins (2 & 3 MP) | wt. % | 32.03 | 8.69 | **72.80** | 42.08 | 0.06 |
| Hexane | wt. % | 8.01 | 0.10 | 5.09 | 40.05 | 0.98 |
| C6 Naphthenes | wt. % | 5.81 | 0.02 | 0.79 | 14.87 | 31.75 |
| Benzene | wt. % | 0 | 0 | 0 | 0 | 0 |
| C7 Paraffins | wt. % | 3.10 | 0.00 | 0.00 | 0.00 | 30.64 |
| C7 Naphthenes | wt. % | 3.70 | 0.00 | 0.00 | 0.00 | 36.58 |
| Total | wt. % | 100 | 100 | 100 | 100 | 100 |
| Reboiler Duty | MMkcal/hr | 11.8 | | | | |
| Condenser Duty | MMkcal/hr | 14.15 | | | | |

**Table 7**

| **Material balance of MDW & partition wall (PW) column of** **Figure 11** | | | | | | |
|---|---|---|---|---|---|---|
| **Stream Description** | **Units** | **Feed** | **Light** | **Isom recycle** | **High-purity** | **Heavy** |
| | | | **Isomerate** | | **Hexane** | **Isomerate** |
| Flowrate | kp/hr | 40000.00 | 17800.00 | 11750.00 | 6450.00 | 4000.00 |
| Composition profile | | | | | | |
| H2 | wt. % | 0 | 0 | 0 | 0 | 0 |
| C3- | wt. % | 0 | 0 | 0 | 0 | 0 |
| C4 Paraffins | wt. % | 0.08 | 0.18 | 0.00 | 0.00 | 0.00 |
| i-Pentane | wt. % | 13.40 | 30.12 | 0.00 | 0.00 | 0.00 |
| n-Pentane | wt. % | 3.90 | 8.76 | 0.00 | 0.00 | 0.00 |
| C5 Naphthenes | wt. % | 0.50 | 1.11 | 0.02 | 0.00 | 0.00 |
| C6 i-Paraffins (2,2 &2,3 MB) | wt. % | 29.51 | **51.78** | 20.69 | 2.34 | 0.00 |
| C6 i-Paraffins (2 & 3 MP) | wt. % | 32.01 | 8.02 | **73.11** | 43.18 | 0.11 |
| Hexane | wt. % | 8.00 | 0.02 | 5.00 | 40.01 | 1.06 |
| C6 Naphthenes | wt. % | 5.80 | 0.00 | 1.17 | 14.47 | 30.83 |
| Benzene | wt. % | 0 | 0 | 0 | 0 | 0 |
| C7 Paraffins | wt. % | 3.10 | 0.00 | 0.00 | 0.00 | 31.00 |
| C7 Naphthenes | wt. % | 3.70 | 0.00 | 0.00 | 0.00 | 37.00 |
| Total | wt. % | 100 | 100 | 100 | 100 | 100 |
| Reboiler Duty | MMkcal/hr | 10.6 | | | | |
| Condenser Duty | MMkcal/hr | 13.27 | | | | |

**Table 8**

| **Comparison of Quality of High Purity Hexane produced by the Solvent Extraction Process vs from Isomerization Unit** | | | |
|---|---|---|---|
| | | Solvent Extraction Process | Four Cut DWC |
| n-hexane | % wt. | | > 40 |
| Sulfur | mq / kq | 1.0 - 5.0 | < 0.5 |
| Benzene | mq / kq | 130 - 240 | < 3.0 |

### Reference Numerals

- 10: Prior art solvent extraction plant
- 12: Extraction column
- 14: Wash column
- 16: Mercaptan removal unit
- 18: Splitter columns
- 20: Splitter columns
- 22: Splitter columns
- 24: Prior art isomerization unit
- 25: Feed
- 26: Recycle gas
- 28: Recycle gas compressor
- 30: Reactor feed-effluent exchanger
- 32: Reactor one
- 34: Reactor two
- 36: Product separator
- 38: Gas
- 40: Compressor
- 42: Stabilizer
- 44: Deisohexanizer (DIH) distillation column
- 46: Prior art plant for producing a purified n-hexane stream
- 48: Deisohexanizer (DIH) distillation column
- 50: Isomerate recycle distillation column
- 52: n-Hexane distillation column
- 54: Light isomerate
- 56: Isomerate process recycle stream
- 58: High purity n-hexane
- 60: Heavy isomerate
- 62: Indicates thermodynamic inefficiency
- 64: Dividing-wall distillation column in accordance with the present invention
- 66: Dividing wall
- 68: First top section of dividing-wall distillation column
- 70: Second top section of dividing-wall distillation column
- 72: Bottom section of dividing-wall distillation column
- 74: Inlet for stable isomerate stream
- 76: Outlet for light isomerate stream
- 78: Outlet for purified n-hexane stream
- 80: Outlet for heavy isomerate stream
- 82: Outlet for an isomerate process recycle stream
- 84: Recycle line for isomerate process recycle stream
- 86, 86': Overhead condenser
- 88, 88', 88", 88‴: Recycle line into distillation column
- 90, 90': Bottom reboiler
- 92: First bottom section of dividing-wall distillation column
- 94: Second bottom section of dividing-wall distillation column
- 96: Top section of dividing-wall distillation column
- 98: First side section of dividing-wall distillation column
- 100: Second side section of dividing-wall distillation column
- 102, 102': Side condenser
- 104: Partition wall
- 106: Horizontal section of partition wall
- 108: Vertical section of partition wall
- 110: Isomerization unit

## Claims

1. A method for producing n-hexane, which comprises the following steps:
a) producing an isomerate stream in an isomerization unit comprising at least one isomerization reactor,
b) feeding the isomerate stream produced in step a) into a dividing-wall distillation column and distilling it,
c) removing from the dividing-wall distillation column four separate streams, namely a light isomerate stream, a heavy isomerate stream, a purified n-hexane stream and an isomerate process recycle stream and
d) recycling the isomerate process recycle stream to at least one of the at least one isomerization reactor of the isomerization unit.

2. The method in accordance with claim 1, wherein the isomerate stream produced in step a) is a stable isomerate stream, which preferably comprises at least 80 wt% of C4-7 hydrocarbons, wherein preferably at least 50 wt%, more preferably at least 60 wt% and most preferably at least 70 wt% of the isomerate stream are branched alkanes, or, wherein the isomerate stream produced in step a) comprises at least 80 wt% of C5-6 hydrocarbons, wherein preferably at least 50 wt%, more preferably at least 60 wt% and most preferably at least 70 wt% of the isomerate stream are branched alkanes.

3. The method in accordance with claim 1 or 2, wherein the purified n-hexane stream removed in step c) from the dividing-wall distillation column has a n-hexane content of at least 30 wt%, preferably of at least 35 wt%, more preferably of 35 to 45 wt%, yet more preferably of at least 40 wt% and most preferably of 40 to 45 wt%, and/or, wherein the purified n-hexane stream removed in step c) from the dividing-wall distillation column has a benzene content of < 3 ppm wt. and a sulfur content of < 0.5 ppm wt.

4. The method in accordance with any of the preceding claims, wherein the light isomerate stream removed in step c) from the dividing-wall distillation column has an octane number of 87 to 89 and comprises at least 80 wt% of branched C5-6 hydrocarbons, wherein preferably at least 70 wt%, more preferably at least 80 wt% and most preferably at least 90 wt% of the light isomerate stream are branched alkanes.

5. The method in accordance with any of the preceding claims, wherein the heavy isomerate stream removed in step c) from the dividing-wall distillation column has an octane number of 82 to 87 and comprises at least 80 wt% of C6+ hydrocarbons, wherein preferably at least 40 wt%, more preferably at least 50 wt% and most preferably at least 60 wt% of the heavy isomerate stream are C7+ hydrocarbons and preferably 10 to 60 wt%, more preferably 20 to 50 wt% and most preferably 20 to 40 wt% of the heavy isomerate stream are C6 cycloalkanes.

6. The method in accordance with any of the preceding claims, wherein the dividing-wall distillation column used in step c) is a top dividing-wall column, wherein the dividing wall of the top dividing-wall column extends from the upper end of the top dividing-wall column over 20 to 80% and preferably over 20 to 70% of the height of the top dividing-wall column at least essentially vertically downwards so that the top dividing-wall distillation column comprises on one side of the dividing wall a first top section, on the opposite side of the dividing wall a second top section and below the dividing wall a bottom section, wherein essentially vertically downwards means that the angle between the dividing wall and the length axis of the top dividing-wall distillation column is at most 20°, preferably at most 10°, more preferably at most 5° and most preferably 0°.

7. The method in accordance with claim 6, wherein the isomerate stream is fed in step b) as side fraction into the first top section of the top dividing-wall distillation column, wherein in step c) the light isomerate stream is removed as overhead fraction from the first top section of the top dividing-wall distillation column, the purified n-hexane stream is removed as overhead fraction from the second top section of the top dividing-wall distillation column, the heavy isomerate stream is removed as bottom fraction from the bottom section of the top dividing-wall distillation column and the isomerate process recycle stream is removed as side fraction from the first top section of the top dividing-wall distillation column.

8. The method in accordance with claim 6, wherein the dividing wall of the top dividing-wall column extends from the upper end of the top dividing-wall column over 5 to 60% and preferably over 10 to 50% of the height of the top dividing-wall column at least essentially vertically downwards, wherein the top dividing-wall column further comprises a partition wall arranged below the dividing wall and comprising an essentially horizontally arranged section and a lower essentially vertically arranged section, wherein the upper essentially horizontally arranged section comprises a first edge and a second edge and the lower essentially vertically arranged section comprises an upper edge and a lower edge, wherein the upper edge of the lower essentially vertically arranged section and the first edge of the upper essentially horizontally arranged section of the partition wall are connected with each other over the whole length of both edges, and wherein the second edge of the upper essentially horizontally arranged section of the partition wall is fluid tightly connected with the outer wall of the top dividing-wall column, wherein essentially vertically downwards means that the angle between the dividing wall and the length axis of the top dividing-wall distillation column is at most 20°, preferably at most 10°, more preferably at most 5° and most preferably 0°, wherein essentially horizontally means that the angle between the dividing wall and the cross-sectional plane of the top dividing-wall distillation column is at most 20°, preferably at most 10°, more preferably at most 5° and most preferably 0°, so that the top dividing-wall distillation column comprises on one side of the dividing wall a first top section, on the opposite side of the dividing wall a second top section, in the volume below the essentially horizontally arranged section of the partition wall extending until the second edge of the essentially vertically arranged section of the partition wall a partitioned section and in the residual volume of the top dividing-wall column a bottom section.

9. The method in accordance with claim 8, wherein the isomerate stream is fed in step b) as side fraction into the first top section of the top dividing-wall distillation column, wherein in step c) the light isomerate stream is removed as overhead fraction from the first top section of the top dividing-wall distillation column, the isomerate process recycle stream is removed as overhead fraction from the second top section of the top dividing-wall distillation column, the heavy isomerate stream is removed as bottom fraction from the bottom section of the top dividing-wall distillation column and the purified n-hexane stream is removed as side fraction from the partitioned section of the top dividing-wall distillation column.

10. The method in accordance with any of claims 1 to 5, wherein the dividing-wall distillation column used in steps b) and c) is a bottom dividing-wall column, wherein preferably the dividing wall of the bottom dividing-wall column extends from the lower end of the bottom dividing-wall column over 10 to 60% and preferably over 20 to 50% of the height of the bottom dividing-wall column at least essentially vertically upwards so that the bottom dividing-wall distillation column comprises on one side of the dividing wall a first bottom section, on the opposite side of the dividing wall a second bottom section and above the dividing wall a top section, wherein essentially vertically upwards means that the angle between the dividing wall and the length axis of the bottom dividing-wall distillation column is at most 20°, preferably at most 10°, more preferably at most 5° and most preferably 0°, wherein preferably the isomerate stream is fed in step b) as side fraction into the first bottom section of the bottom dividing-wall distillation column, wherein in step c) the light isomerate stream is removed as overhead fraction from the top section of the bottom dividing-wall distillation column, the heavy isomerate stream is removed as bottom fraction from the first bottom section of the middle dividing-wall distillation column, the purified n-hexane stream is removed as bottom fraction from the second bottom section of the middle dividing-wall distillation column and the isomerate process recycle stream is removed as side fraction from the second bottom section of the bottom dividing-wall distillation column.

11. The method in accordance with any of claims 1 to 5, wherein the dividing-wall distillation column used in steps b) and c) is a middle dividing-wall column, wherein preferably the dividing wall of the middle dividing-wall column extends, seen from the bottom to the top of the middle dividing-wall distillation column, from a point being located at 20 to 50% of the distance from the bottom to the top of the middle dividing-wall distillation column to a point being located at 70 to 90% of the distance from the bottom to the top of the middle dividing-wall distillation column at least essentially vertically downwards so that the middle dividing-wall distillation column comprises above the dividing wall a top section, below the dividing wall a bottom section, on one side of the dividing wall a first middle section and on the opposite side of the dividing wall a second middle section, wherein essentially vertically downwards means that the angle between the dividing wall and the length axis of the middle dividing-wall distillation column is at most 20°, preferably at most 10°, more preferably at most 5° and most preferably 0°.

12. A plant for producing n-hexane, which comprises:
i) an isomerization unit comprising at least one isomerization reactor, wherein the isomerization unit comprises an inlet for a hydrocarbon feed stream and an outlet for an isomerate stream and the at least one isomerization reactor comprises an inlet and an outlet, and
ii) a dividing-wall distillation column, which comprises one inlet and four outlets, wherein the inlet of the dividing-wall distillation column is connected with the outlet for an isomerate stream of the isomerization unit, wherein a first outlet is for removing from the dividing-wall distillation column a light isomerate stream, a second outlet is for removing from the dividing-wall distillation column a heavy isomerate stream, a third outlet is for removing from the dividing-wall distillation column a purified n-hexane stream and a fourth outlet is for removing from the dividing-wall distillation column an isomerate process recycle stream, wherein the dividing-wall distillation column further comprises a recycle line, which fluidly connects the fourth outlet for removing from the dividing-wall distillation column an isomerate process recycle stream and the inlet of at least one of the at least one isomerization reactor.

13. The plant in accordance with claim 12, wherein the recycle line directly leads into the inlet of at least one of the at least one isomerization reactor, or, wherein the recycle line leads to a mixer, to which also a feed line to the at least one isomerization reactor leads, for mixing the isomerate process recycle stream and the feed stream of the at least one isomerization reactor, wherein the mixer further comprises an outlet line, which directly leads into the inlet of at least one of the at least one isomerization reactor.

14. The plant in accordance with any of claims 32 to 34, wherein the dividing-wall distillation column is a top dividing-wall column or a bottom dividing-wall column or a middle dividing-wall column.

15. The plant in accordance with claim 14, wherein the dividing-wall distillation column is a top dividing-wall column, wherein the dividing wall of the top dividing-wall column extends from the upper end of the top dividing-wall column perpendicularly downwards over 5 to 60% and preferably over 10 to 50% of the height of the top dividing-wall column at least essentially vertically downwards, wherein the top dividing-wall column further comprises a partition wall arranged below the dividing wall and comprising an essentially horizontally arranged section and a lower essentially vertically arranged section, wherein the upper essentially horizontally arranged section comprises a first edge and a second edge and the lower essentially vertically arranged section comprises an upper edge and a lower edge, wherein the upper edge of the lower essentially vertically arranged section and the first edge of the upper essentially horizontally arranged section of the partition wall are connected with each other over the whole length of both edges, and wherein the second edge of the upper essentially horizontally arranged section of the partition wall is fluid tightly connected with the outer wall of the top dividing-wall column, so that the top dividing-wall distillation column comprises on one side of the dividing wall a first top section, on the opposite side of the dividing wall a second top section, in the volume below the essentially horizontally arranged section of the partition wall extending until the second edge of the essentially vertically arranged section of the partition wall a partitioned section and in the residual volume of the top dividing-wall column a bottom section.
